# EUROPÄISCHE PATENTSCHRIFT

(11) **EP 0 194 579 B1**
(45) Veröffentlichungstag und Bekanntmachung des Hinweises auf die Patenterteilung: **03.04.1991**
(21) Anmeldenummer: 86102932.0
(22) Anmeldetag: 05.03.1986
(51) Int. Cl.: C07D 213/30, C07D 213/42, C07D 213/26, C07D 213/55, A61K 31/44

(54) **Neue 3-Pyridylmethylnaphthylderivate, Verfahren zu ihrer Herstellung und ihre Verwendung als Arzneimittel**
3-Pyridylmethylnaphthyl derivatives, process for their preparation and their use as medicaments
Dérivés 3-pyridylméthylnaphtyle, leur procédé de préparation et leur utilisation comme médicament

(30) Priorität: 13.03.1985 DE 3508903
(43) Veröffentlichungstag der Anmeldung: 17.09.1986
(73) Patentinhaber: HOECHST AKTIENGESELLSCHAFT, 65926 Frankfurt am Main (DE)
(72) Erfinder: Lau, Hans-Hermann, Dr., D-6232 Bad Soden am Taunus (DE); Bartmann, Wilhelm, Dr., D-6232 Bad Soden am Taunus (DE); Beck, Gerhard, Dr., D-6000 Frankfurt am Main 1 (DE); Wess, Günther, Dr., D-6455 Erlensee (DE)

(56) Entgegenhaltungen:
- EP-A- 0 050 957
- EP-A- 0 073 663

## Beschreibung

3-substituierte Pyridinderivate sind Hemmer der Thromboxan-Synthetase (T. Tanouchi, M. Kawamura, I. Ohyama, I. Kajiwara, Y. Iguchi, T. Okada, T. Miyamoto, K. Taniguchi, M. Hayashi, K. lizuka, M. Nakazawa, J. Med. Chem. 24 , 1149 (1981).

Das Enzym Thromboxan-Synthetase katalysiert innerhalb des Arachidonsäuremetabolismus die Umwandlung der Prostaglandinendoperoxide (PGH₂ bzw. PGG₂) in Thromboxan A₂ (TXA₂). TXA₂ ist biologisch hoch aktiv: es induziert die Aggregation der Blutplättchen und wirkt außerdem auf die glatte Muskulatur stark konstriktiv. Es spielt eine wesentliche Rolle bei der Haemostase, bei pathologischen Situationen mit erhöhter Tendenz zu Gefäßkrämpfen und/oder Thrombose. Ferner wirkt TXA₂ in vitro und in vivo stark kontrahierend auf die Bronchialmuskulatur (B. Samuelsson, Angew. Chem. 95 , 848 (1983).

Die neuen 3-Pyridylmethylnaphthylcarbonsäuren, deren Carbonsäurederivate und 3-Pyridylmethylnaphthylmethanole, die in der vorliegenden Erfindung beschrieben werden, zeichnen sich durch eine spezifische Hemmung der Thromboxan-Synthetase aus.

Sie eignen sich daher zur Prophylaxe oder Behandlung von Krankheiten mit gestörter (erhöhter) Thrombocytenaggregationsneigung, sowie bei pathologisch erhöhten Thromboxanspiegeln, wie sie bei Ischämie, Angina pectoris, thromboembolischen Erkrankungen, Atherosklerose, Koronarkrämpfen, Arrhythmien, cerebralen ischämischen Anfällen, Migräne und anderen vaskulären Kopfschmerzen, Myokardinfarkt, Hypertension, Atmungsstörungen wie Asthma und Apnoe, Entzündungskrankheiten sowie mikrovaskulären Komplikationen bei Diabetes mellitus gefunden werden. Außerdem sind die Verbindungen geeignet, die Proliferation von Tumorzellen zu verlangsamen bzw. zu verhindern.

3-Pyridylmethylnaphthalin- bzw. 3-Pyridylcarbonylnaphthalin-Derivate sind bereits aus EP-A2-0 073 663 bekannt. Bei diesen Verbindungen sind Naphthyl- und Pyridylrest über eine -CH₂- bzw. -CO-Gruppe miteinander verknüpft.

Gegenstand der vorliegenden Erfindung sind neue 3-Pyridylmethylnaphthylcarbonsäuren, deren Carbonsäurederivate und 3-Pyridylmethylnaphthylmethanole der Formel I, 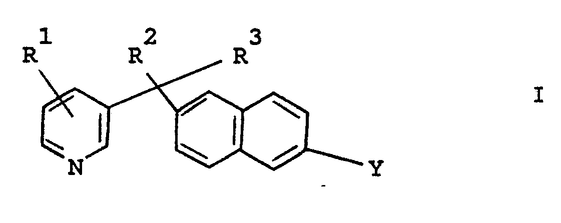 sowie die physiologisch verträglichen Säureadditionssalze.

In der allgemeinen Formel I bedeuten:
R' Wasserstoff oder in 2-, 4-, -5- oder 6-Stellung Halogen, einen geradkettigen oder verzweigten aliphatischen Kohlenwasserstoffrest mit 1-6 C-Atomen, einen Cycloalkylrest mit 3-7 C-Atomen, einen Arylalkylrest oder einen Arylrest, welche im Kern 1-3fach substituiert sein können mit Halogen, Alkyl und/oder Alkoxy mit je 1-6 C-Atomen oder einen Hydroxy- oder einen Alkoxyrest mit 1-6 C-Atomen,
R² einen Hydroxyrest, Halogen, einen Alkoxyrest mit 1-6 C-Atomen oder einen Phenoxyrest, der im Kern 1-3fach substituiert sein kann mit Halogen, Trifluormethyl, Alkyl und/oder Alkoxy mit je 1-6 C-Atomen,
R³ Wasserstoff, einen Cycloalkylrest mit 3-7 C-Atomen, einen geradkettigen oder verzweigten Alkylrest mit 1-8 C-Atomen, einen geradkettigen oder verzweigten ungesättigten aliphatischen Kohlenwasserstoffrest mit bis zu 8 C-Atomen und bis zu 3-Doppel- oder Dreifachbindungen, einen Phenylrest, der im Kern 1-3fach mit Halogen, Trifluormethyl, Hydroxy, Alkyl und/oder Alkoxy mit je 1-6 C-Atomen substituiert sein kann, einen 2-, 3- oder 4-Pyridylrest, der in 4- oder 5-Stellung mit C, -C₄.-Alkyl substituiert sein kann,
Y einen Rest der Formel 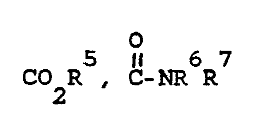 oder CH₂0H, wobei bedeuten
R⁵ Wasserstoff, einen geradkettigen oder verzweigten Alkylrest mit bis zu 8 Kohlenstoffatomen, einen geradkettigen oder verzweigten ungesättigten aliphatischen Kohlenwasserstoffrest mit bis zu 6 Kohlenstoffatomen, einen cycloaliphatischen Kohlenwasserstoffrest mit 3 bis 7 Kohlenstoffatomen, einen araliphatischen Kohlenwasserstoffrest mit 7 bis 10 Kohlenstoffatomen oder ein physiologisch verträgliches Metall-, NH₄- oder ein Ammoniumion, das sich von einem primären, sekundären oder tertiären Amin ableitet, oder ein Tetraalkylammoniumion,
R⁶ Wasserstoff, einen geradkettigen oder verzweigten Alkylrest mit 1-6 C-Atomen, einen cycloaliphatischen Kohlenwasserstoffrest mit 3-7 C-Atomen, einen araliphatischen Kohlenwasserstoffrest mit 7-10 C-Atomen, der im Kern 1-3fach substituiert sein kann mit Halogen, Trifluormethyl, Alkyl und/oder Alkoxy mit je 1-6 C-Atomen, oder einen Phenylrest, der im Kern 1-3fach substituiert sein kann mit Halogen, Trifluormethyl, Alkyl und/oder Alkoxy mit je 1-6 C-Atomen,
R⁷ Wasserstoff, einen geradkettigen oder verzweigten Alkylrest mit 1 - 6 C-Atomen oder
R⁶ und R⁷ zusammen eine (CH₂)ₙ Gruppe mit n = 3-6 oder eine -(CH₂)p-Z-(CH₂)p -Gruppe mit p = 2 oder 3, wobei
Z Sauerstoff oder N-R⁸ ist, wobei
R⁸ Wasserstoff oder einen geradkettigen oder verzweigten Alkylrest mit 1-6 C-Atomen bedeutet.

Unter den Substituenten R' sind besonders bevorzugt: Wasserstoff oder in der 4- oder 5-Stellung Halogen, ein geradkettiger oder verzweigter Alkylrest mit 1-6 C-Atomen, insbesondere C₁-C₄-Alkyl oder einen cycloaliphatischen Kohlenwasserstoffrest mit 5-7 C-Atomen, insbesondere C₅-C₇-Cylcoalkyl.

Unter den Substituenten R² sind besonders bevorzugt: Hydroxy, Halogen, Alkoxy mit 1-4 C-Atomen oder ein Phenoxyrest, der im Kern bevorzugt unsubstituiert ist, oder 1-2fach mit C₁-C₃-Alkyl, insbesondere Methyl oder Ethyl, mit C₁-C₃-Alkoxy, insbesondere Methoxy oder Ethoxy, mit Halogen oder mit Trifluormethyl substituiert ist.

Unter den Substituenten R³ sind besonders bevorzugt:

Wasserstoff, geradkettiges oder verzweigtes Alkyl mit 1-6 C-Atomen, ein Cycloalkylrest mit 5-7 C-Atomen, insbesondere C₅-C₇-Cycloalkyl oder ein Phenylrest, der im Kern unsubstituiert oder 1-2fach mit Cₗ-C₃-Alkyl, insbesondere Methyl oder Ethyl, Hydroxy, Cₗ-C₃-Alkoxy, insbesondere Methoxy oder Ethoxy, Halogen oder mit Trifluormethyl substituiert ist, oder 3-Pyridyl, das in 4- oder 5-Position mit Ci-C₃-Alkyl, insbesondere Methyl oder Ethyl substituiert sein kann.
Y hat bevorzugt die Bedeutung 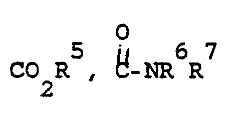 oder CH₂0H, wobei für R⁵, R⁶ und R⁷ vorzugsweise folgende Bedeutungen in Frage kommen:
   R⁵ Wasserstoff, geradkettiges oder verzweigtes Alkyl mit bis zu 6 C-Atomen, insbesondere C₁-C₄-Alkyl, ein geradket tiger oder verzweigter ungesättigter, aliphatischer Kohlenwasserstoffrest mit bis zu 4 C-Atomen, insbesondere C₂-C₄-Alkenyl, ein cycloaliphatischer Kohlenwasserstoffrest mit 5-7 C-Atomen, insbesondere Cs-C₇-Cycloalkyl, ein araliphatischer Kohlenwasserstoffrest mit 7-10 C-Atomen, insbesondere Phenethyl oder Benzyl, oder ein physiologisch verträgliches Metall-, NH₄-oder ein Ammoniumion, das sich von einem primären, sekundären oder tertiären Amin ableitet, insbesondere:
   Wasserstoff, Methyl, Ethyl, n-Butyl, n-Pentyl, n-Hexyl, n-Heptyl, 2-Propyl, 2-Butyl, 2-Pentyl, 3-Hexyl, 2-Methylpropyl, 2-Methylbutyl, 4,4-Dimethylpentyl, 5,5-Dimethylhexyl, Cyclopentyl, Cyclohexyl, Cycloheptyl, Methylammonium, Dicyclohexylammonium, Tris-(hydroxymethyl)-methylammonium.
   R⁶ Wasserstoff, geradkettiges oder verzweigtes Alkyl mit bis zu 6 C-Atomen, insbesondere C₁-C₄-Alkyl, ein cycloaliphatischer Kohlenwasserstoffrest mit 5-7 C-Atomen, insbesondere Cyclopentyl und Cyclohexyl, ein araliphatischer Kohlenwasserstoffrest mit 7-10 C-Atomen, insbesondere Benzyl und 2-Phenylethyl oder ein Phenylrest.
   R⁷ Wasserstoff, geradkettiges oder verzweigtes Alkyl mit 1 bis 4 C-Atomen.
   R⁶ und R⁷ können zusammen bevorzugt bedeuten:
      eine -(CH₂)ₙ-Gruppe mit n = 4 oder 5 oder eine -(CH₂)₂-O-(CH₂)₂-Gruppe.

Besonders bevorzugt sind Verbindungen der Formel I, worin R¹ Wasserstoff oder Ci-C₂-Alkyl oder Phenyl in 4-Stellung, R² Hydroxy, C₁-C₂-Alkoxy oder Chlor, R³ Wasserstoff oder Cₗ-C₂-Alkyl und Y die COOR^{s-}Gruppe mit R⁵ = H oder C₁-C₄-Alkyl bedeuten, sowie die physiologisch verträglichen Säureadditionssalze.

Ferner umfaßt die Erfindung Säureadditionssalze der beschriebenen Verbindungen mit anorganischen oder organischen Säuren, z.B. Chlorwasserstoffsäure, Bromwasserstoffsäure, Schwefelsäure, Phosphorsäure, Salpetersäure, Essigsäure, Propionsäure, Oxalsäure, Malonsäure, Glykolsäure, Bernsteinsäure, Maleinsäure, Fumarsäure, Weinsäure, Citronensäure, Benzoesäure oder Zimtsäure.

Gegenstand der Erfindung ist ferner ein Verfahren zur Herstellung von Verbindungen der Formel I, das dadurch gekennzeichnet ist, daß man
a) eine Verbindung der Formel II 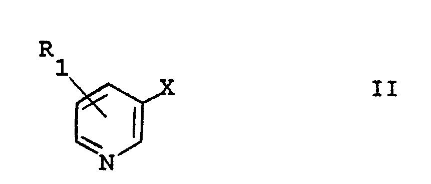 worin R¹ die zur Formel I angegebene Bedeutung hat und X Chlor, Brom oder Jod bedeutet, selektiv in der 3-Position metalliert und anschließend mit einer Verbindung der Formel III 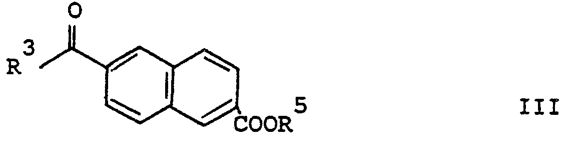 worin R³ die zur Formel I genannte Bedeutung hat und R⁵ C₁-C₈-Alkyl bedeutet, umsetzt zu einer Verbindung der Formel I, worin R² Hydroxy und Y die Gruppe COOR⁵ mit R⁵ = C₁-C₈-Alkyl darstellt,
   α) gegebenenfalls die erhaltene Verbindung selektiv halogeniert zu einer Verbindung der Formel I, worin R² Halogen und Y die Gruppe -CO0R⁵ und R⁵ = C₁-C₈-Alkyl bedeutet
   β) gegebenenfalls eine Verbindung der Formel I, worin R² Halogen und Y die Gruppe -COOR^{S} mit R^{S} = C₁-C₈-Alkyl darstellt, mit einer Verbindung der Formel IV 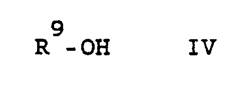 worin R⁹ Ci-C₆-Alkyl oder einen Phenylrest darstellt, der im Kern 1-3fach substituiert sein kann mit Halogen, Trifluormethyl, Alkyl- und/oder Alkoxy mit 1-6 C-Atomen, in Gegenwart einer Base umsetzt zu einer Verbindung der Formel I, worin R² die Gruppe -OR⁹ und Y die Gruppe -COOR⁵ mit R^{S} = Ci-Cs-Alkyl darstellt, oder
b) eine Verbindung der Formel V 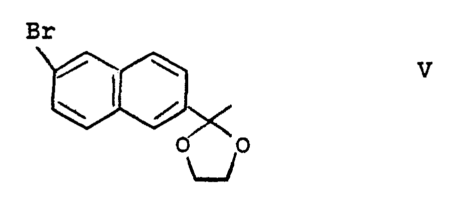 selektiv in der 2-Position metalliert und anschließend mit einer Verbindung der Formel VI 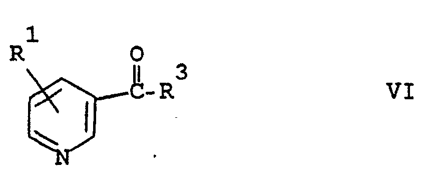 worin R¹ und R³ die zur Formel 1 angegebenen Bedeutungen haben, zu einer Verbindung der Formel VII 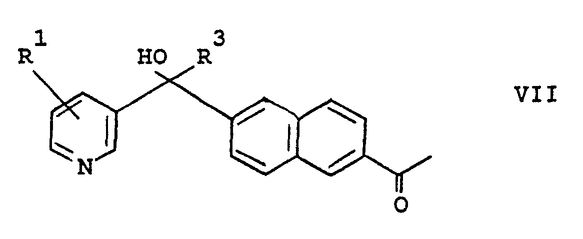 umsetzt, und
   i) eine Verbindung der Formel VII, worin R¹ und R³ die zur Formel I angegebene Bedeutung haben, R³ jedoch nicht Wasserstoff ist, oxydiert zu einer Verbindung der Formel I, worin R² die Hydroxy- und Y die Carboxygruppe darstellen,
   ii) in einer Verbindung der Formel VII, worin R¹ die zur Formel I genannte Bedeutung hat und R³ Wasserstoff bedeutet, zunächst die Hydroxygruppe mit einer Schutzgruppe schützt, anschließend die Verbindung oxidiert und schließlich die Schutzgruppe wieder abspaltet, wobei man zu einer Verbindung der Formel I gelangt, worin R² die Hydroxygruppe, R³ Wasserstoff udn Y die carboxygruppe darstellen,
   iii) eine Verbindung der Formel VII, worin R¹ die zur Formel I genannte Bedeutung hat und R³ primäres oder sekundäres Alkyl bedeuten, zunächst dehydratisiert und anschließend katalytisch hydriert zu einer Verbindung der Formel VIII 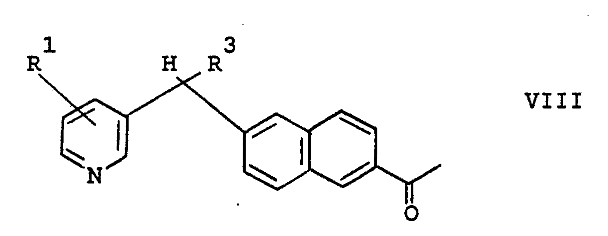 worin R¹ und R³ die oben genannten Bedeutungen haben, oder eine Verbindung der Formel VII selektiv halogeniert zu einer Verbindung der Formel IX 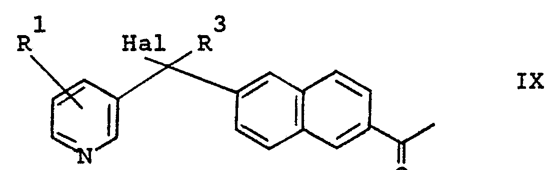 worin R' und R³ die zur Formel I genannten Bedeutungen haben und Hal Chlor, Brom oder Jod darstellt, und die erhaltene Verbindung entweder reduziert zu einer Verbindung der Formel VIII 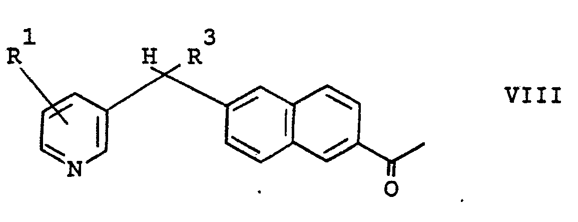 oder mit einer Verbindung der Formel IV 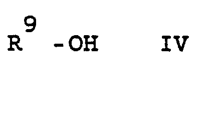 worin R⁹ C₁-C₆-Alkyl oder einen Arylrest darstellt, der im Kern 1-3fach substituiert sein kann mit Halogen, Trifluormethyl, Alkyl und/oder Alkoxy mit 1-6 C-Atomen, in Gegenwart einer Base zu einer Verbindung der Formel X 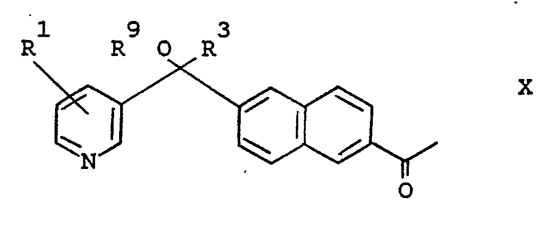 umsetzt, und
      eine erhaltene Verbindung der Formel VIII, IX oder X oxidiert zu einer Verbindung der Formel I, worin R¹ und R³ die oben zur Formel I genannten Bedeutungen haben, Y die Carboxygruppe darstellt und R² Halogen, bzw. die Gruppe OR⁹ bedeutet,
      und gegebenenfalls eine nach Verfahren a) oder b) erhaltene Verbindung der Formel I, worin Y Carboxyl oder Alkoxycarbonyl darstellt, in die entsprechenden Ester, Amide oder Salze überführt, oder reduziert zu einer Verbindung der Formel I, worin Y die Gruppe -CH₂OH bedeutet.

Das im erfindungsgemäßen Verfahren (b) als Ausgangsmaterial eingesetzte 2-Bromnaphthalinderivat der Formel V kann nach literaturbekannten Verfahren aus 6-Acetyl-2-bromnaphthalin synthetisiert werden, das wiederum nach R.B. Girdler, P.H. Gore, J.A. Hoskins, J. Chem. Soc. (C), 1966, 518 erhalten werden kann.

3-Acetylpyridine der allg. Formel VI können nach den in Heteroxyclic Compounds, Pyridine and its derivatives, part 4, p. 123 f, 1964 (New York, London, Sidney) beschriebenen Verfahren, insbesondere aber auch der Methode nach J. Cason, Chem. Reviews, 40 , 15 (1947) hergestellt werden.

Die Synthese von 3-halogenierten Pyridinderivaten (Verfahren a) der allgemeinen Formel II gelingt u.a. nach den in Bull. Soc. Chim. Fr., 1972, 2466 und Bull. Soc. Chim. Fr., 1976, 530 beschriebenen Verfahren.

6-Formyl-2-napthoesäureester der Formel III kann nach dem in DOS 23 63 416 beschriebenen Verfahren synthetisiert werden. Weitere Ausgangsverbindungen der allgemeinen Formel III mit R³ = Wasserstoff können in Analogie zu literaturbekannten Methoden aus den 6-Formyl-2-naphthoesäureestern hergestellt werden.

0-substituierte Hydroxylamine sind nach dem in Houben-Weyl, Bd X/1, S. 1192 und W.B. Lutz, J. Org. Chem., 36 , 3835 (1971) beschriebenen Verfahren aus N-Hydroxyphthalimid herstellbar.

Methoden zur Darstellung von metallierten Produkten aus Verbindungen der allgemeinen Formel II bzw. V sind beschrieben für die Lithiumderivate (U. Schöllkopf in Houben-Weyl, Bd. XIII/1, S. 3f und S. 87f), für die Magnesiumderivate (K. Nützel in Houben-Weyl, Bd. XIII/2, S 47f) und für die Titanderivate (B. Weidmann, D. Seebach, Angew. Chem. 95 , 12 (1983).

Für die Darstellung von Verbindungen der Formel I mit R² = Halogen bzw. von Verbindungen der Formel IX eignen sich be sonders die Umsetzungen der entsprechenden Hydroxyverbindungen der Formeln I bzw. VII mit Thionylchlorid (s. Houben-Weyl, Bd. V/3, S. 862) bzw. mit Triphenylphosphin/ Tetrachlormethan (E. I. Snyder, J. Org. Chem., 37 , 1466 (1972)).

Die Ketone der Formel VII, VIII und X können in einer Haloform-Reaktion mit Chlor, Brom oder Jod in Gegenwart einer starken Base wie z.B. Natrium- oder Kaliumhydroxyd zu Verbindungen der Formel I oxidiert werden, in denen Y eine Carboxylgruppe bedeutet.

Nach literaturbekannten Verfahren lassen sich aus Verbindungen der Formel I mit Y = Carboxyl oder Alkoxycarbonyl die entsprechenden Ester, Amide bzw. Salze herstellen.

Die Reduktion der Ester- bzw. Säurefunktion in Verbindungen der Formel I zu Verbindungen der Formel I mit Y = CH₂-OH gelingt u.a. mit komplexen Metallhydriden wie Lithiumalanat oder Natriumboranat in einem geeigneten inerten Lösungsmittel.

Die Verbindungen der allgemeinen Formel I zeigen eine spezifische Hemmung der Thromboxan-Synthetase und können deshalb als Arzneimittel zur Prophylaxe oder Behandlung von Krankheiten mit gestörter, d.h. erhöhter Aggregationsneigung der Thrombocyten, sowie bei pathologisch erhöhten Thromboxanspiegeln, wie sie bei Ischämie, Angina pectoris, thromboembolischen Erkrankungen, Atherosklerose, Koronarkrämpfen, Arrhythmien, cerebralen ischämischen Anfällen, Migräne und anderen vaskulären Kopfschmerzen, Myokardinfarkt, Hypertension, Atmungsstörungen wie Asthma oder Apnoe, Entzündungskrankheiten sowie mikrovaskulären Komplikationen bei der Diabetes mellitus gefunden werden, verwendet werden. Die erfindungsgemäßen Verbindungen beeinflussen Erkrankungen mit erhöhten Thromboxanspiegeln in verschiedenen Organen günstig, z.B. im Nieren- oder im Magen-Darm-Bereich bei Colitis oder bei "inflammatory bowel disease". Die Verbin dungen sind zudem geeignet, die Proliferation von Tumorzellen zu verlangsamen oder sogar zu verhindern.

Die Verbindungen sind in Dosen von 0,01 mg/kg bis 10 mg/kg wirksam. Die verabreichte Einzeldosis kann zwischen 1 mg bis 500 mg liegen. Bei oraler Applikation liegt die bevorzugte Tagesdosis zwischen 1 mg und 1 g.

Die Metaboliten der Arachidonsäure sind an einer Reihe von physiologischen und pathophysiologischen Prozessen beteiligt. Bei der Regulation des Tonus der Blutgefäße und der Thrombocytenaggregation sind Prostacyclin (PG1₂) und Thromboxan A₂ (TXA₂) von wesentlicher Bedeutung. Prostacyclin, das bevorzugt in den Endothelzellen der Blutgefäße aus Prostaglandinendoperoxid H₂ (PGH₂) gebildet wird, bewirkt eine Vasodilation und verhindert gleichzeitig die Aggregation der Thrombocyten. Die Umwandlung von PGH₂ in Prostacyclin wird durch die Prostacyclin-Synthetase katalysiert. Der physiologische Gegenspieler von Prostacyclin ist Thromboxan A₂, das vornehmlich in den Blutplättchen aus PGH₂ synthetisiert wird. Diese Reaktion wird durch das Enzym Thromboxan-Synthetase katalysiert. TXA₂ bewirkt eine Aggregation der Blutplättchen und führt zu einer Vasokonstriktion. Es ist bislang der stärkste bekannte Vasokonstriktor im menschlichen Organismus (s. A. G. Herman, P. M. Vonhoutte, H. Denolin, A. Goossens, Cardiovascular Pharmacology of the Prostaglandins, Raven Press, New York, 1982). Störungen im Gleichgewicht zwischen Prostacyclin und Thromboxan A₂ führen zu pathophysiologischen Situationen. Bei gleichbleibenden PGI₂-Spiegeln führt eine Erhöhung des Thromboxanspiegels daher zu einer Blutplättchenaggregation und zu Gefäßspasmen sowie zu einer erhöhten Anfälligkeit gegenüber Atherothrombose (Lancet 1977 , 479; Science 1976 , 1135; Amer. J. Cardiology 41 , 787 (1978); Lancet 1977 , 1216;).

Bei der experimentellen Atherosklerose ist die Bildung von PGI₂ inhibiert bei gleichzeitig erhöhter Thromboxan Az-Bildung (Prostaglandins 14 , 1025 und 1035 (1977)). Aus diesem Grunde wird TXA₂ mit verschiedenen Anginen, myocardialer Infarktbildung, plötzlichem Herztod und Schlaganfällen in Verbindung gebracht (Thromb. Haemostasis 38 , 132 (1977); Platelets, Prostaglandins and Cardiovascular System, Florenz, Februar 1984).

Ein weiteres Gebiet, auf dem eine Störung des PGI₂/TXA₂-Gleichgewichtes als ein beitragender Faktor angesehen wird, ist die Migräne. Der Migränekopfschmerz ist mit Veränderungen des intra- und extracerebralen Blutflusses verbunden, insbesondere mit einer vor dem Auftreten des Kopfschmerzes erfolgenden Reduzierung des cerebralen Blutflusses und nachfolgender Dilatation in beiden Gefäßbereichen während der Kopfschmerzphase. Thrombocyten von Migränepatienten besitzen eine größere Neigung zur Aggregation als diejenigen von normalen Individuen (J. clin. Pathol. 24 , 250 (1971); J. Headache, 17 , 101 (1977); Lancet 1978 , 501).

Bei Patienten mit Diabetes mellitus wird ein Ungleichgewicht zwischen Prostacyclin und Thromboxan A₂ als Ursache für die mikrovaskulären Komplikationen angesehen. Thrombocyten von Diabetespatienten bilden erhöhte Mengen an TXA₂ und Malondialdehyd (Symposium "Diabetes and Thrombosis - Implications for Therapy", Leeds, Großbritannien, April 1979). Es wurde außerdem gezeigt, daß bei Ratten mit experimentell erzeugter Diabetes die vaskuläre PGI₂-Bildung gehemmt ist, während die TXA₂-Synthese in den Plättchen erhöht ist (IV. lnt. Prostaglandin Conference, Washington DC, Mai 1979).

Nichtsteroidale Antiinflammatorika hemmen die Cyclooxygenase, die die Umwandlung der Arachidonsäure in PGH₂ über PGG₂ katalysiert. Sie greifen daher sowohl in die Biosynthese des Thromboxan A₂ als auch in die des Prostacyclins ein. Wertvoller wären somit Verbindungen, die spezifisch die Bildung von Thromboxan A₂ durch Hemmung der Thromboxan-Synthetase blockieren und gleichzeitig die Bildung von Prostacyclin nicht beeinflussen.

### Versuchsbericht

### Die biochemische und pharmakologische Aktivität wurde in folgenden Testsystemen bestimmt:

### 1. Hemmung der Arachidonsäure-induzierten Aggregation von Humanthrombocyten in vitro

Gesund erscheinenden wännlichen und weiblichen Freiwilligen, die im vorangegangenen Zeitraum von 10 Tagen keine Medikamente eingenommen haben, wird durch vorsichtige Kanülierung der Antekubitalvene Blut entnommen, das sofort mit Natriumcitrat (a d 0.38 %) stabilisiert wird. Durch 15 minütiges Zentrifugieren bei 140 x g erhält man im Überstand plättchenreiches Plasma (PRP), dessen Thrombocytengehalt im Bereich 2.5 - 3.5x 10⁸/ ml (Coulter-Counter) liegen sollte. Die Plättchenaggregation wird optisch durch Messung der Lichttransmission in einem Born'schen Aggregometer verfolgt. Das Gesamtvolumen des Testansatzes beträgt 0.25 ml. Die Vorinkubationszeit mit Prüfpräparat beträgt 10 min bei 37° C, die Aggregationsinduktion erfolgt danach mit 2 x 10⁻⁴ M Arachidonsäure. Das Prüfpräparat wird in der Regel in fünf verschiedenen Konzentrationen in PRP aus drei verschiedenen Sendern getestet. Aus den jeweiligen maximalen Aggregationsamplituden werden Dosis-Wirkungskurven erstellt und graphisch ICso-Werte (ICso ist die Konzentration,die eine 50 %ige Hemmung der Arachidonsäure-induzierten Aggregation bewirkt) bestimmt. Die Messungen erfolgen im Zeitraum von 1-6 Stunden nach der Blutentnahme.

Von den erfindungsgemäßen Verbindungen wurden nach der oben beschriebenen Methode folgende ICso-Werte für die Hemmung der Arachidonsäure-induzierten Aggregation von Humanthrombocyten in vitro ermittelt :

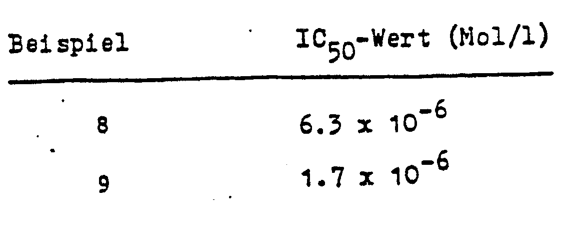

### 2. Thrombin induzierte TXA₂-Freisetzung in plättchenreichem Humanplasma in vitro

Gesund erscheinenden männlichen und weiblichen Freiwilligen, die im vorangegangenen Zeitraum von 10 Tagen keine Medikamente eingenommen haben, wird durch vorsichtige Kanülierung der Antekubitalvene Blut entnommen, das sofort mit Natriumzitrat (a d. 0,38 %) stabilisiert wird. Durch 15 minütiges Zentrifugieren bei 140 x g erhält man im Überstand plättchenreiches Plasma (PRP), dessen Thrombozytengehalt im Bereich 2,5 - 3,5 10⁸ /ml liegen sollte. Durch erneutes Zentrifugieren (10 min bei 2000 x g) werden die Thrombozyten sedimentiert und anschließend in Tyrode-Lösung re-solubilisiert (ca. 7.10⁷ Plättchen / ml, Gesamtvolumen pro Messung 0,5 ml). Nach Zugabe von Testsubstanz wird 10 min bei 37° C inkubiert, anschließend 7,2 10-⁷ M Arachidonsäure und 0,5 E Thrombin zugesetzt und 30 min bei 37° C inkubiert. Hierauf wird im Eisbad abgestoppt und nach Zugabe von Tracer und TXB₂-spezifischem Antikörper (NEN, Dreieich) der TXB_{z}-Gehalt radioimmunologisch bestimmt (TXA₂ ist unter den Versuchsbedingungen instabil und daher nicht meßbar. Stattdessen wird das stabile Hydrolyseprodukt TXB₂ gemessen). Meßgröße ist der relative TXB₂-Gehalt in den Thrombozyten-Inkubationen aus zwei bis drei verschiedenen Spendern mit und ohne (= 100 %) Prüfsubstanz.

Nach der oben beschriebenen Methode wurden von den erfindungsgemäßen Verbindungen z.B. folgende Werte für die TXB₂-Freisetzung nach Gabe von 10-⁶ bzw. 10-⁷ Mol/I Testsubstanz ermittelt :

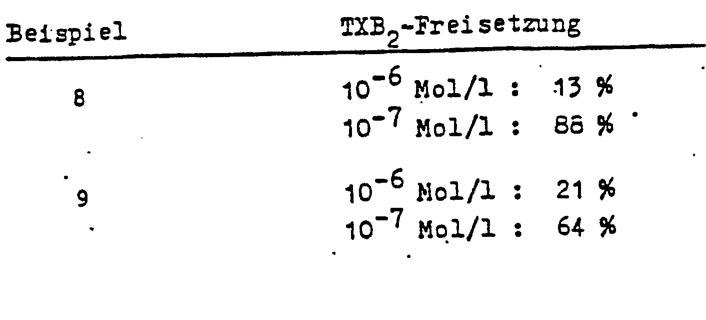

### 3. Hemmung der Laser-induzierten Thrombose

Die Untersuchungen der erfindungsgemäßen Verbindungen im Laser-induzierten Thrombosemodell erfolgt an männlichen oder weiblichen Sprague-Dawley-Ratten mit einem Körpergewicht von ca 200 g. Die zu untersuchenden Tiere werden mit 0.1 mg Atropinum sulf. sol. s.c. prämediziert und mit 100 mg Ketaminhydrochlorid und 4 mg Xylazin pro kg Körpergewicht i.p. anästhetisiert. Zur Untersuchung dienen Arteriolen des Mesenteriums mit einem Durchmesser von 12 - 25 µm. Während der Messung erfolgt eine Hyperfundierung des exponierten Mesenteriums mit erwärmter phys. NaCI (37° C) oder eine Überschichtung mit entgastem Paraffinöl.
Der Strahl des 4 W Argon-Lasers (Fa. Spectra Physics, Darmstadt, FRG) wird mittels einer Strahladaptions-und -justieranlage (Fa. BTG, München, FRG) koaxial in den invertierten Strahlengang eines Mikroskopes (ICM 405, LD-Epilan 40/0.60; Fa. Zeiss, Oberkochen, FRG) eingebracht. Die benutzte Wellenlänge beträgt 514.5 nm mit einer Energie oberhalb des Objektives von 40 mW. Die Expositionszeit pro Einzelschuß dauert 1/15 sec. Der Durchmesser des effektiven Laserstrahls auf dem Gefäß beträgt 10 um, bei wiederholter Exposition erfolgt der nächste Schuß jeweils 5 u.m stromaufwärts unmittelbar an der Gefäßwand. Alle Meßvorgänge werden per Videokamera (Sony, Trinicon-Röhre) aufgenommen und auf einem Recorder (Sony, U-matic 3/4) gespeichert. Im Durchlichtverfahren des gleichen Mikroskops wird bei kleiner Vergrößerung (LD-Epilan 8/0.20) ein Überblicksbild der zu untersuchenden Endstrombahn geliefert. Ein Videoanalyser sowie ein Korrelator dient zur Bestimmung der Strömungsgeschwindigkeit in der untersuchten Arteriole.

Die Testsubstanzen wurden in 0.9 % Natriumchlorid-Lösung (enthielt 1,1 Carboxymethylzellulose oder in entsprechenden Lösungsvermittlern) den Versuchstieren in verschiedenen Dosierungen oral eine Stunde vor Versuchsbeginn verabreicht: Kontrolltiere wurden in entsprechender Weise, aber ohne Testsubstanzen, behandelt. Die Untersuchungen wurden randomisierend als Doppelblindstudie durchgeführt.

### Auswertung :

Es wird die Anzahl der Schüsse gezählt, um einen definierten Thrombus zu induzieren. Die Frequenz der Laserblitze beträgt eine Läsion alle 2 Minuten, wobei alle während des Beobachtungszeitraumes gebildeten Thromben von einer Mindestgröße von 1/4 Gefäßradius ausgezählt und vermessen werden. Die statistische Auswertung der Versuchsergebnisse erfolgt mit Hilfe des x^{Z-}Testes (L. Cavalli-Sforza, Biometrie, Stuttgart, 1969, S 49f).

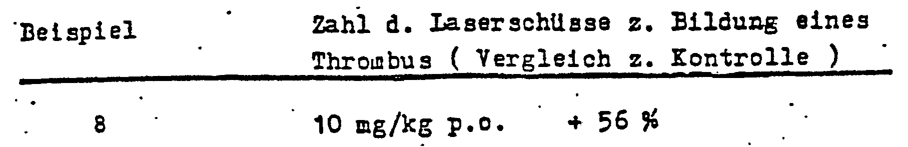

Die Verbindungen der Formel I blockieren spezifisch die Bildung von Thromboxan A₂ durch Hemmung der Thromboxan-Synthetase ohne Beeinflussung der Prostacyclinbildung und eignen sich daher zur Vorbeugung oder zur Behandlung der oben aufgeführten Erkrankungen, die auf eine Hemmung der Thromboxan-Synthetase ansprechen.

Die Erfindung betrifft daher ferner die Verwendung der Verbindungen der Formel 1 und von deren Salzen zur Verwendung als Arzneimittel sowie pharmazeutische Präparate auf Basis der erfindungsgemäßen Verbindungen

Die Verbindungen der Formel werden in verschiedenen Dosierungsformen verabreicht, z.B. oral In Form von Tabletten, Kapseln oder Flüssigkeiten, rektal in Form von Suppositorien, parenteral, subkutan oder intramuskulär, wobei intravenöse Verabreichung in Notsituationen bevorzugt wird.

Die erfindungsgemäßen Verbindungen der Formel I können als freie Basen oder in Form ihrer physiologisch unbedenklichen anorganischen oder organischen Säureadditionssalze zur Anwendung kommen. Die freien Basen und Säureadditionssalze können In Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Ethanol, Ethylenglykol oder Glycerin, in Triacetin, in Alkohol-Acetaldehyd-diacetalgemischen, Ölen wie z.B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon zur Anwendung gelangen.

Als Zubereitungen können die üblichen galenischen Infusions- oder Injektionslösungen und Tabletten sowie örtlich anwendbare Zubereitungen wie Cremes, Emulsionen, Suppositorien oder Aerosole in Frage kommen.

### Herstellung von Ausgangsverbindungen:

### Beispiel 1:

### 2-Brom-6-naphthylmethylketonethylenacetal

19 g (76,3 mMol) 2-Brom-6-acetylnaphthalin, 10,7 ml (11.76 g, 0.19 Mol) Ethylenglykol und 250 mg p. Toluolsulfonsäure werden in 150 ml Toluol am Wasserabscheider unter Rückfluß erhitzt, bis kein Wasser mehr abgeschieden wird (ca. 1 Stunde). Die Toluollösung wird mit ges. Natriumbicarbonatlösung extrahiert, die wiederum zweimal mit Essigester reextrahiert wird. Die vereinigten organischen Phasen werden getrocknet und im Vakuum eingeengt. Aus dem Rückstand werden durch Umkristallisieren aus Petrolether 16.02 g (72 %) 2-Brom-6-naphthylmethylketonethylenacetal erhalten. Schmp. 80-2 C.
¹H-NMR (CDCI₃, 60 MHz) δ = 1,7 (s, 3H; CH₃). 3,6-4,2 (m, 4H; -OCH₂-CH₂O-), 7,4-8,0 (m, 6H; arom. H)

### Beispiel 2a:

### 2-Acetyl-6[1-hydroxy-1-(3-pyridyl)methyl]-naphthalin

Unter Argon werden bei -70 C zu einer Lösung von 16,1 g (55 mMol) 2-Brom-6-naphthylmethylketo- nethylenacetal in 120 ml abs. Ether unter Rühren 36,1 ml (57,75 mMol) 1,6 M n-Butyllithium in Hexan zugetropft. Anschließend läßt man auf n °C kommen und rührt eine Stunde nach. Bei -30 ° C werden dann 6,19 g (57,75 mMol, 5,45 ml) Nicotinaldehyd in 15 ml abs. Ether zugetropft. Danach läßt man innerhalb von einer Stunde auf Raumtemperatur kommen und rührt eine weitere Stunde nach. Nach Ansäuern mit 2N HCI bis PH 1 wird über Nacht gerührt. Nach Trennung der Phase wird die wäßrige Phase einmal mit Ether extrahiert, die Etherphase verworfen und die wäßrige Phase mit Natriumbicarbonatlösung neutralisiert, die dann mit Essigester extrahiert wird. Nach Trocknen und Einengen wird der Rückstand aus Isopropanol umkristallisiert. Erhalten werden 9.2 g (60 %) 2-Acetyl-6[i-hydroxy-1-(3-pyridyl)methyl] -naphthalin. Schmp. 157-9 C.
¹H-NMR (CDCl₃, 60 MHz) 8 = 2,7 (s, 3H; CH₃), 3,2 (b, 1 H; OH), 6,0 (s, 1 H; CH), 7,0-8,6 (m, 10H; arom. H).

### Beispiel 2b:

In Analogie zu 2a wird ebenfalls 2-Acetyl-6-[1-Hy droxyl-1-(3-pyridyl)ethyl]naphthalin erhalten. Schmp. 224-7 ° C. ¹H-NMR (DMSO-d₆, 60 MHz) δ = 2,0 (s, 3H; CH₃), 2,7 (s, 3H; C(0)CH₃), 6,1 (s, 1 H; OH), 7,1-8,7 (m, 10H; arom. H).

### Beispiel 2c:

In Analogie zu 2a wird 2-Acetyl-6-[1-hydroxy-l-phenyl-1-(3-pyridyl}methyl]naphthalin erhalten. Schmp. 117-120 ° C.
¹H-NMR (CDCl₃, 60 MHz) δ = 2,7 (s, 3H; CH₃), 3,7 (b, 1 H; OH), 7,0-8,6 (m, 15H; arom. H).

### Herstellung von Endprodukten

### Beispiel 3a:

### 6-[1-Hydroxy-1-(3-pyridyl)methyl]-2-naphthoesäure methylester

9,17 g (33,1 mMol) 2-Acetyl-6-[1-hydroxy-l-(3-pyridyl) methyl] naphthalin und 13,8 ml (99,3 mMol) abs. Triethylamin in 200 ml abs. Dichlormethan werden unter Rühren bei 0 ° C mit 8,4 ml (66,2 mMol) Trimethylchlorsilan versetzt. Nach Stehenlassen über Nacht bei Raumtemperatur wird eingeengt, in Natriumbicarbonatlösung aufgenommen und mit Essigester extra hiert. Nach Trocknen und Einengen wird der Rückstand (12,4 g) in 35 ml Dioxan gelöst und bei 0 bis 10 ° C zu einer Lösung von 14.2 g (0.355 Mol) Natriumhydroxid in 70 ml Wasser,zu der vorher 5,5 ml (106,5 mMol, 17 g) Brom getropft wurden, zugegeben. Nach Stehen über Nacht bei Raumtemperatur werden 10 ml Aceton zugesetzt, 30 Minuten nachgerührt, zweimal mit Essigester extrahiert, die wäßrige Phase mit 2N HCI auf PH2 eingestellt und eingeengt. Der Rückstand wird anschließend mit Isopropanol ausgekocht und eingeengt. Der verbleibende Rückstand wird in 400 ml 1 N methanolischer Salzsäure aufgenommen und über Nacht stehengelassen bei Raumtemperatur. Dann wird eingeengt, in Natriumcarbonatlösung aufgenommen und mit Essigester extrahiert. Nach Trocknen und Einengen werden 4,4 g Rohprodukt erhalten, aus denen durch Chromatographie über Kieselgel (Essigester) 3,63g (37 %) 6-[1-Hydroxy-1-(3-pyridyl)methyl]-2-naphthoesäuremethylester isoliert werden.
R_{F}= (Essigester-Methanol 8:1): 0,38; Schmp. 130-5 ° C. ¹H-NMR (CDCl₃, 60 MHz) δ = 2,8 (b, 1H; OH), 3,95 (s, 3H; CH₃), 6,0 (s, 1H; CH), 7,1-8,1 und 8,4-8,7 (m, 10H; arom. H).

### Beispiel 3b:

In Analogie zu 3a wird aus 2-Acetyl-6-[1-hydroxy-1-(3-pyridyl)ethyl]naphthalin, ohne daß die Hydroxygruppe als Trimethylsilylether geschützt werden muß, 6-[1-Hydroxy-1-(3-pyridyl)ethyl]-2-naphthoesäureme- thylester erhalten. R_{F} (Essigester-Methanol 8:1): 0.4; Schmp.: 190-2 C. ¹H-NMR (CDCl₃, 60 MHz) 0 = 2,0 (s, 3H; C-CH₃); 2,5 (b, 1 H; OH), 3,9 (s, 3H; OCH₃), 7,0-8,7 (m, 10H; arom. H).

### Beispiel 3c:

In Analogie zu 3a wird ohne intermediären Schutz der Hydroxygruppe als Trimethylsilylether aus 2-Acetyl-6-[1-hydroxy-1-phenyl-1-(3-pyridyl)methyl]-naphthalin 6-[1-Hydroxy-1-phenyl-1-(3-pyridyl)methyl]-2-naphthoe säuremethylester hergestellt. R_{F} (Essigester-Methanol): 0,53.
¹H-NMR (CDCl₃, 60 MHz) δ = 3,7 (b, 1 H; OH), 3,95 (s, 3H; CH₃), 7,1-8,7 (m, 15H: arom. H).

### Beispiel 4:

### 6-[1-Chlor-1-(3-pyridyl)methyl]-2-naphthoesäuremethyl ester

0,3 g (1,02 mMol) 6-[1-Hydroxy-1-(3-pyridyl)methyl]-2-naphthoesäuremethylester und 0,349 g (1,33 mMol) Triphenylphosphin werden in 3 ml abs. Tetrachlormethan gelöst und drei Stunden unter Rückfluß erhitzt. Anschliessend wird mit 2NHCI extrahiert, die wäßrige Phase mit-Natriumbicarbonatlösung neutralisiert und mit Essigester reextrahiert. Nach Trocknen und Einengen im Vakuum wird der Rückstand mit Cyclohexan-Essigester 1:1 über Kieselgel chromatographiert. Erhalten werden 73 mg (23 %) 6-[1-Chlor-1-(3-pyridyl)methyl]-2-naphthoesäuremethylester als Öl. R_{F} (Essigester): 0,45.
¹H-NMR (CDCI₃, 60 MHz) δ = 3,95 (s, 3H; CH₃), 6,3 (s, 1 H; CH), 7,2-8,7 (m, 10H; arom. H).

### Beispiel 5:

### 6-[1-Methoxy-1-(3-pyridyl)methyl]-2-naphthoesäuremethyl ester

52 mg (0,17 mMol) 6-[1-Chlor-1-(3-pyridyl)methyl]-2-naphthoesäuremethylester und 8 mg (0,18 mMol) Natriumhydrid-Disp. (55 %) werden in 4 ml abs. Methanol 6 Std. unter Rückfluß erhitzt. Nach Einengen der Reaktionsmischung wird in Natriumbicarbonatlösung aufgenommen und mit Essigester extrahiert. Nach Trocknen und Einengen der Essigesterphase im Vakuum wird der Rückstand mit Essigester über Kieselgel chromatographiert. Erhalten werden 48,2 mg (94 %) 6-[1-Methoxy-1-(3-pyridyl) methyl]-2-naphthoesäure- methylester. R_{F} (Essigester-Cyclohexan 1:1): 0,17.
¹H-NMR (CDCl₃, 60 MHz) δ = 3,4 (s, 3H; Ether-CH₃), 3,95 (s, 3H; Ester-CH₃), 5,45 (s, 1 H; CH), 7,1-8,7 (m, 10H; arom. H).

### Beispiel 6:

### 6-[1-Hydroxy-l-(4-methyl-3-pyridyl)methyl]-2-naphthoesäure-Hydrochlorid

1,26 g (3,76 mmol) 6-[1-Hydroxy-1-(4-methyl-3-pyridyl)methyl]-2-naphthoesäureisopropylester werden in 25 ml 2N HCI und 20 ml konz. HCI drei Stunden unter Rückfluß erhitzt, mit Aktivkohle versetzt und heiß filtriert. Beim Abkühlen kristallisiert das Hydrochlorid der Säure aus. Nach Absaugen, Nachwaschen mit wenig Aceton und Trocknen werden 0,93 g (75 %) 6-[1-Hydroxy-1-(4-methyl-3-pyridyl)methyl]-2-napt hoesäure-Hydrochlorid erhalten. Schmp.: 264-7° C (Zers.)
¹H-NMR (D₂₀, 60 MHz) δ = 2,33 (s,3H; CH₃), 6,25 (s, 1H; CH), 7,2-8,9 (m, 9H; arom. H).

### Beispiel 7:

Analogie zu 6 wird aus 6-[1-Hydroxy-1-(3-pyridyl) methyl]-2-naphthoesäuremethylester das Hydrochlorid der 6-[1-Hydroxy-1-[3-pyridyl)methyl]-2-naphthoesäure hergestellt. Schmp. 227-9 ° C (Zers.).
¹H-NMR (D₂0, 60 MHz) 8 = 6,2 (s, 1H; CH), 7,3-8,9 (m, 10H; arom. H).

### Beispiel 8:

### 6-[-1-Hydroxy-1-(4-methyl-3-pyridyl)methyl]-2-naphthoe säure-isopropylester

3,25 g (18,9 mMol) 3-Brom-4-methylpyridin werden unter Argon in 50 ml abs. Äther vorgelegt und bei -70° C langsam mit 12,4 ml (19,8 mMol) 1,6 M n-Butyllithiumlösung in Hexan versetzt. Es wird 30 min. nachgerührt und bei -70° C 5,43 g (20,8 mMol) Triisopropoxytitanchlorid in 20 ml abs. Äther zugetropft. Man läßt das Reaktionsgemisch auf 0 ° C kommen und tropft es bei dieser Temperatur zu einer Lösung von 2,7 g (12,6 mMol) 6-Formyl-2-naphthoesäuremethylester in 50 ml abs. Äther und 20 ml abs. Tetrahydrofuran. Das Reaktionsgemisch wird bei Raumtemperatur bis zum vollständigen Umsatz des Aldehyds gerührt (^{~} 24 h).

Es wird mit 50 ml Äther verdünnt und mit 2 N HCI bis pH 1 angesäuert. Nach zweimaliger Extraktion mit Äther wird die wäßrige Phase mit ges. Natriumbicarbonatlösung neutralisiert. Der Niederschlag wird abgesaugt und mit Isopropanol und Essigester ausgekocht. Die organischen Phasen werden eingeengt, und der Rückstand wird aus Essigester umkristallisiert. Die Mutterlauge wird mit Essigester-Methanol (8:1) über Kieselgel chromatographiert. Erhalten werden insgesamt 2,3 g (54 %) 6-[1-Hydroxy-1-(4-methyl-3-pyridyl)-methyl]-2-naphthoesäureisopropylester.

Schmo. 180-3° C.

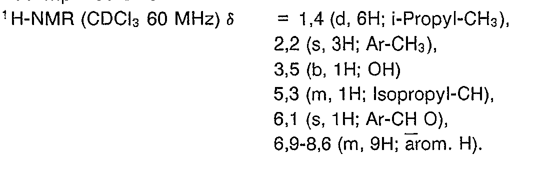

### Beispiel 9:

### 1-(6-Hydroxymethyl-2-naphthyl)-1-(4-methyl-3-pyridyl)methanol

0,3 g (0,9 mMol) 6-[1-Hydroxy-1-(4-mothyl-3-pyridyl)methyl]-2-naphthoesäureisopropylester in 5 ml abs. Tetrahydrofuran werden bei 0° C zu 38 mg (1 mMol) Lithiumalanat in 1 ml abs. Tetrahydrofuran gegeben und dann bei Raumtemperatur gerührt (24 h). Nach Zugabe von weiteren 40 mg (1 mMol) Lithiumalanat wird 24 h bei Raumtemperatur nachgerührt. Überschüssiges Lithiumalanat wird mit 2N HCI zersetzt und die Lösung mit Natriumbicarbonatlösung neutralisiert. Es wird mit Essigester extrahiert, eingeengt und der Rückstand mit Essigester-Methanol 4 : 1 über Kieselgel chromatographiert. Erhalten werden 0,16 g (65%) --(6-Hydroxymethyl-2-naphthyl)-1-(4-methyl-3-pyridyl)methanol.

Schmp. 151-3 C

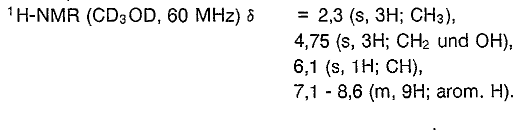

## Patentansprüche (Patentansprüche für folgende(n) Vertragsstaat(en) : BE CH DE FR GB IT LI LU NL SE)

1. Verbindungen der Formel I 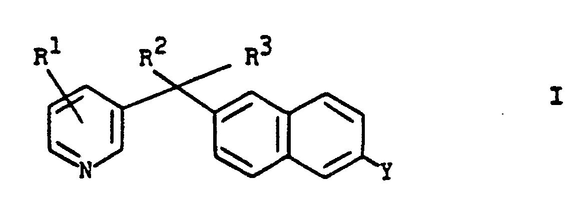 worin bedeuten:
R' Wasserstoff oder in 2-, 4-, 5- oder 6-Stellung Halogen, einen geradkettigen oder verzweigten Alkylrest mit 1 bis 6 C-Atomen, einen Cycloalkylrest mit 3-7 C-Atomen, einen Phenyl-C₁-C₂-alkyl oder einen Phenylrest, welche im Kern 1-3fach substituiert sein können mit Halogen, Alkyl und/oder Alkoxy mit je 1-6 C-Aomen, oder einen Hydroxy- oder einen Alkoxyrest mit 1-6 C-Atomen
R² einen Hydroxyrest, Halogen, einen Alkoxyrest mit 1-6 C-Atomen oder einen Phenoxyrest, der im Kern 1-3fach substituiert sein kann mit Halogen, Trifluormethyl, Alkyl und/oder Alkoxy mit je 1-6 C-Atomen
R³ Wasserstoff, einen Cycloalkylrest mit 3-7 C-Atomen, einen geradkettigen oder verzweigten Alkylrest mit 1-8 C-Atomen, einen geradkettigen oder verzweigten ungesättigten aliphatischen Kohlenwasserstoffrest mit bis zu 8 C-Atomen und bis zu 3 Doppel-oder Dreifachbindungen, einen Phenylrest, der im Kern 1-3fach mit Halogen, Trifluormethyl, Hydroxy, Alkyl und/oder Alkoxy mit je 1-6 C-Atomen substituiert sein kann, oder einen 2-, 3- oder 4-Pyridylrest, der in 4- oder 5-Position mit C₁-C₄-Alkyl substituiert sein kann.
Y einen Rest der Formel 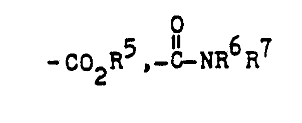 oder -CH₂OH, wobei bedeuten
R⁵ Wasserstoff, einen geradkettigen oder verzweigten Alkylrest mit bis zu 8 Kohlenstoffatomen, einen geradkettigen oder verzweigten ungesättigten aliphatischen Kohlenwasserstoffrest bis zu 6 Kohlenstoffatomen, einen cycloaliphatischen Kohlenwasserstoffrest mit 3-7 Kohlenstoffatomen, einen araliphatischen Kohlenwasserstoffrest mit 7-10 Kohlenstoffatomen oder ein physiologisch verträgliches Metall-, NH₄. oder ein Ammoniumion, das sich von einem primären, sekundären oder tertiären Amin ableitet, oder ein Tetraalkylammoniumion,
R⁶ Wasserstoff, einen geradkettigen oder verzweigten Alkylrest mit 1 - 6 C-Atomen, einen cycloaliphatischen Kohlenwasserstoffrest mit 3-7 C-Atomen, einen araliphatischen Kohlenwasserstoffrest mit 7-10 C-Atomen, der im Kern 1-3fach substituiert sein kann mit Halogen, Trifluormethyl, Alkyl und/oder Alkoxy mit je 1-6 C-Atomen, oder einen Phenylrest,der im Kern 1-3fach substituiert sein kann mit Halogen, Trifluormethyl, Alkyl und/oder Alkoxy mit je 1-6 C-Atomen,
R⁷ Wasserstoff, einen geradkettigen oder verzweigten Alkylrest min 1-6 C-Atomen oder
R⁶ und R⁷ zusammen eine -(CH₂)ₙ-Gruppe mit n = 3-6 oder eine -(CH₂)ₚ-Z-(CH₂)ₚ-Gruppe mit p = 2 oder 3, wobei
Z Sauerstoff oder N-R^{s} ist, wobei bedeutet
R⁸ Wasserstoff oder einen geradkettigen oder verzweigten Alkylrest mit 1-6 C-Atomen, sowie deren physiologisch verträgliche Säureadditionssalze.

2. Verbindungen der Formel I gemäß Anspruch 1 worin bedeuten:
R¹ Wasserstoff oder in 4- oder 5-Stellung Halogen, C₁-C₄-Alkyl oder C₅-C₇-Cycloalkyl,
R² Hydroxy, Halogen, C₁-C₄-Alkoxy, unsubstituiertes Phenoxy oder Phenoxy 1-2-fach substituiert mit Methyl, Ethyl, Methoxy, Ethoxy, Halogen oder Trifluormethyl,
R³ Wasserstoff, C₁-C₆-Alkyl, C₅-C₇-Cycloalkyl, unsubstituiertes Phenyl oder Phenyl, das 1-2fach substituiert ist mit Methyl, Ethyl, Hydroxy, Methoxy, Ethoxy, Halogen oder Trifluormethyl, oder 3-Pyridyl, das in 4- oder 5-Stellung mit Methyl oder Ethyl substituiert sein kann
Y 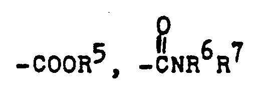 oder CH₂0H, wobei R⁵ Wasserstoff, Ci-C₄.-Alkyl, C₂-C₄-Alkonyl, C₅-C₇-Cycloalkyl, Phenethyl, Benzyl oder ein physiologisch verträgliches Metall-, NH₄- oder substituiertes NH₄-lon darstellt,
R⁶ Wasserstoff, C₁-C₄-Alkyl, Cyclopentyl, Cyclohexyl, Phenyl, Benzyl oder Phenethyl darstellt, und R⁷ Wasserstoff oder C₁-C₄-Alkyl, oder R⁶ und R⁷ zusammen eine -(CH₂)ₙ-Gruppe mit n = 4 oder 5 oder eine(-CH₂)₂-O-(CH₂)₂-Gruppe darstellen, sowie deren physiologisch verträgliche Säureadditionssalze.

3. Verbindungen gemäß Anspruch 1, dadurch gekennzeichnet, daß in Formel I R¹ Wasserstoff, C₁-C₂-Alkyl oder Phenyl jeweils in 4-Stellung, R² Hydroxy, C₁-C₂-Alkoxy oder Chlor, R³ Wasserstoff oder C₁-C₂-Alkyl und Y die Gruppe COOR^{s} mit R⁵ = Wasserstoff oder C₁-C₄-Alkyl bedeuten.

4. Arzneimittel, gekennzeichnet durch einen Gehalt an einer Verbindung gemäß Anspruch 1 sowie pharmazeutisch üblichen Hilfs- und Trägerstoffen.

5. Eine Verbindung gemäß Anspruch 1 zur Verwendung als Arzneimittel.

6. Verfahren zur Herstellung von Verbindungen der Formel I gemäß Anspruch 1
sowie von deren pysiologisch verträglichen Säureadditionssalzen,
dadurch gekennzeichnet, daß man
a) eine Verbindung der Formel II 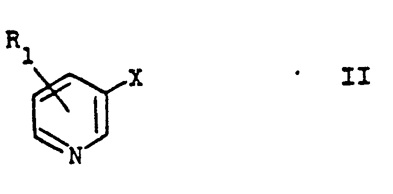 worin R¹ die zur Formel I angegebene Bedeutung hat und X Chlor, Brom oder Jod bedeutet, selektiv in der 3-Position metalliert und anschließend mit einer Verbindung der Formel III 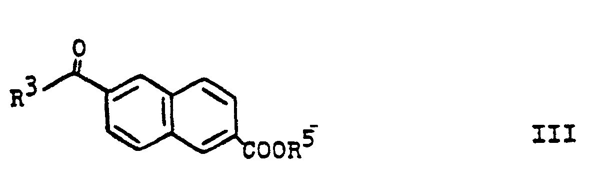 worin R³ die zur Formel genannte Bedeutung hat und R⁵ C₁-C₈-Alkyl bedeutet, umsetzt zu einer Verbindung der Formel I, worin R² Hydroxy und Y die Gruppe COOR^{s} mit R⁵ = C₁-C₈-Alkyl darstellt,
α) gegebenenfalls die erhaltene Verbindung selektiv halogeniert zu einer Verbindung der Formel I worin R² Halogen und Y die Gruppe -COOR^{s} mit R⁵ = C₁-C₈-Alkyl bedeutet,
β) gegebenenfalls eine Verbindung der Formel worin R² Halogen und Y die Gruppe -COOR⁵ mit R⁵ = C₁-C₈-Alkyl darstellt, mit einer Verbindung der Formel IV 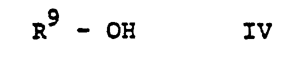 worin R⁹ C₁-C₆-Alkyl oder einen Phenylrest darstellt, der im Kern 1-3fach substituiert sein kann mit Halogen, Trifluormethyl,Alkyl- und/oder Alkoxy mit 1-6 C-Atomen, in Gegenwart einer Base umsetzt zu einer Verbindung der Formel I, worin R² die Gruppe -OR⁹ und Y die Gruppe -COOR^{S} mit R⁵ = C₁-C₈-Alkyl darstellt, oder
b) eine Verbindung der Formel V 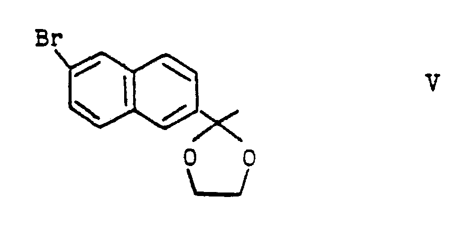 selektiv in der 2-Position metalliert und anschließend mit einer Verbindung der Formel VI 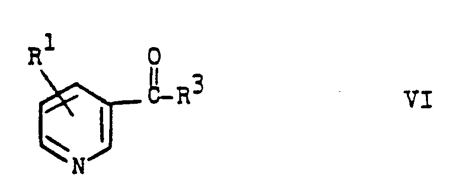 worin R¹ und R³ die zur Formel angegebenen Bedeutungen haben, zu einer Verbindung der Formel VII 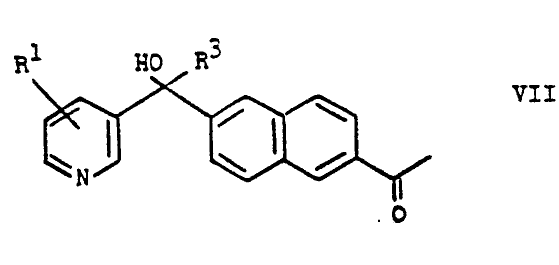 umsetzt, und
i) eine Verbindung der Formel VII, worin R¹ und R³ die zur Formel I angegebene Bedeutung haben, R³ Jedoch nicht Wasserstoff ist, oxydiert zu einer Verbindung der Formel I, worin R² die Hydroxy- und Y die Carboxygruppe darstellen,
ii) in einer Verbindung der Formel VII, worin R¹ die zur Formel I angegebene Bedeutung hat und R³ Wasserstoff bedeutet, zunächst die Hydroxygruppe mit einer Schutzgruppe schützt, anschließend die Verbindung oxidiert und schließlich die Schutzgruppe wieder abspaltet, wobei man zu einer Verbindung der Formel I gelangt, worin R² die Hydroxygruppe, R³ Wasserstoff und Y die Carboxygruppe darstellen,
iii) eine Verbindung der Formel VII, worin R¹ die zur Formel I genannte Bedeutung hat und R³ primäres oder sekundäres Alkyl bedeuten, zunächst dehydratisiert und anschließend katalytisch hydriert zu einer Verbindung der Formel VIII 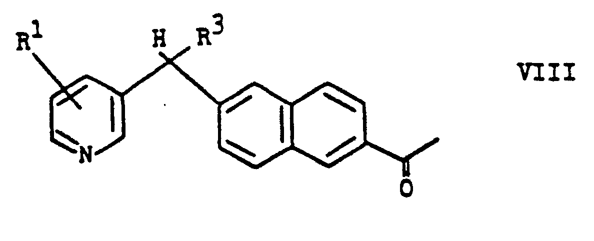 worin R¹ und R³ die oben genannten Bedeutungen haben, oder
eine Verbindung der Formel VII selektiv halogeniert zu einer Verbindung der Formel IX 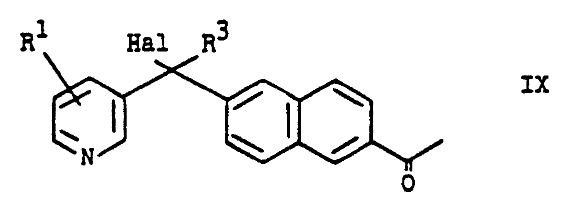 worin R¹ und R³ die zur Formel I genannten Bedeutungen haben und Hal Chlor, Brom oder Jod darstellt, und die erhaltene Verbindung entweder reduziert zu einer Verbindung der Formel VIII 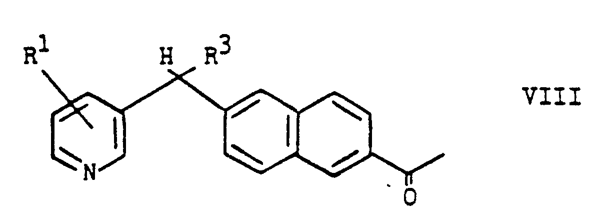 oder mit einer Verbindung der Formel IV 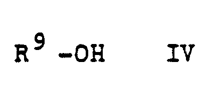 worin R⁹ C₁-C₆-Alkyl oder einen Arylrest darstellt, der im Kern 1-3fach substituiert sein kann mit Halogen, Trifluormethyl Alkyl und/oder Alkoxy-mit 1-6 C-Atomen, in Gegenwart einer Base zu einer Verbindung der Formel X 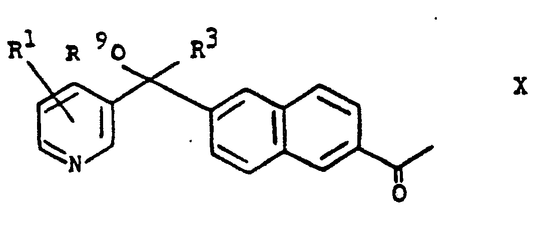 umsetzt, und
eine erhaltene Verbindung der Formel VIII, IX oder X oxidiert zu einer Verbindung der Formel I, worin R¹ und R³ die oben zur Formel I genannten Bedeutungen haben, Y die Carboxygruppe darstellt und R² Halogen, bzw. die Gruppe OR⁹ bedeutet, und gegebenenfalls eine nach Verfahren a) oder b) erhaltene Verbindung der Formel l, worin Y Carboxyl oder Alkoxycarbonyl darstellt, in die entsprechenden Ester, Amide oder Salze überführt, oder reduziert zu einer Verbindung der Formel I, worin Y die Gruppe -CH₂OH bedeutet, und gegebenenfalls eine erhaltene Verbindung der Formel in die Säureadditionssalze überführt.

## Patentansprüche (Patentansprüche für folgende(n) Vertragsstaat(en) : AT)

1. Verfahren zur Herstellung von Verbindungen der Formel 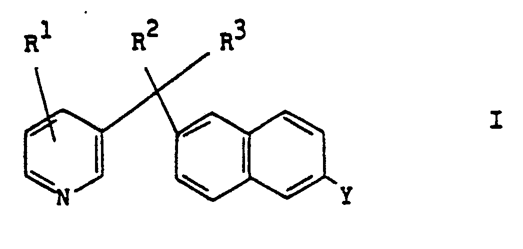 worin bedeuten:
R¹ Wasserstoff oder in 2-, 4-, -5- oder 6-Stellung: Halogen, einen geradkettigen oder verzweigten Alkylrest mit 1 bis 6 C-Atomen, einen Cycloalkylrest mit 3-7 C-Atomen, einen Phenyl-C1-C2-alkyl- oder einen Phenylrest, welche im Kern 1-3fach substituiert sein können mit Halogen, Alkyl und/oder Alkoxy mit je 1-6 C-Atomen oder einen Hydroxy- oder einen Alkoxyrest mit 1-6 C-Atomen
R² einen Hydroxyrest, Halogen, einen Alkoxyrest mit 1-6 C-Atomen oder einen Phenoxyrest, der im Kern 1-3fach substituiert sein kann mit Halogen, Trifluormethyl, Alkyl und/oder Alkoxy mit je 1-6 C-Atomen
R³ Wasserstoff, einen Cycloalkylrest mit 3-7 C-Atomen, einen geradkettigen oder verzweigten Alkylrest mit 1-8 C-Atomen, einen geradkettigen oder verzweigten ungesättigten aliphatischen Kohlenwasserstoffrest mit bis zu 8 C-Atomen und bis zu 3 Doppel-oder Dreifachbindungen, einen Phenylrest, der im Kern 1-3fach mit Halogen, Trifluormethyl, Hydroxy, Alkyl und/oder Alkoxy mit je 1-6 C-Atomen substituiert sein kann, oder einen 2-, 3- oder 4-Pyridylrest, der in 4- oder 5-Position mit C₁-C₄-Alkyl substituiert sein kann
Y einen Rest der Formel 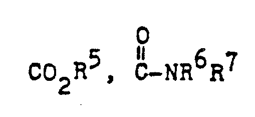 oder CH₂OH, wobei bedeuten
R⁵ Wasserstoff, einen geradkettigen oder verzweigten Alkylrest mit bis zu 8 Kohlenstoffatomen, einen geradkettigen oder verzweigten ungesättigten aliphatischen Kohlenwasserstoffrest mit bis zu 6 Kohlenstoffatomen, einen cycloaliphatischen Kohlenwasserstoffrest mit 3 bis 7 Kohlenstoffatomen, einen araliphatischen Kohlenstoffrest mit 7 bis 10 Kohlenstoffatomen oder ein physiologisch verträgliches Metall-, NH₄ oder ein Ammoniumion, das sich von einem primären, sekundären oder tertiären Amin ableitet, oder ein Tetraalkylammoniumion,
R⁶ Wasserstoff, einen geradkettigen oder verzweigten Alkylrest mit 1-5 C-Atomen, einen cycloaliphatischen Kohlenwasserstoffrest mit 3-7 C-Atomen, einen araliphatischen Kohlenwasserstoffrest mit 7-10 C-Atomen, der im Kern 1-3fach substituiert sein kann mit Halogen, Trifluormethyl, Alkyl und/oder Alkoxy mit je 1-6 C-Atomen, oder einen Phenylrest, der im Kern 1-3fach substituiert sein kann mit Halogen, Trifluormethyl, Alkyl und/oder Alkoxy mit je 1-6 C-Atomen,
R⁷ Wasserstoff, einen geradkettigen oder verzweigten Alkylrest mit 1-6 C-Atomen oder
R⁶ und R⁷ zusammen eine -(CH₂)ₙ-Gruppe mit n = 3-6 oder eine -(CH₂)ₚ-Z-(CH₂)ₚ-Gruppe mit p = 2 oder 3, wobei
Z Sauerstoff oder N-R^{s} ist, wobei bedeutet
R⁸ Wasserstoff oder einen geradkettigen oder verzweigten Alkylrest mit 1-6 C-Atomen, sowie von deren physiologisch verträglichen Säureadditionssalzen, dadurch gekennzeichnet, daß man
a) eine Verbindung der Formel II 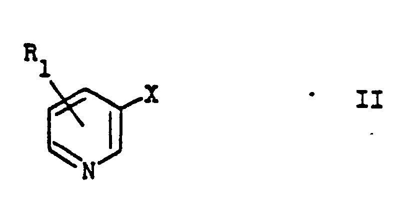 worin R¹ die zur Formel angegebene Bedeutung hat und X Chlor, Brom oder Jod bedeutet, selektiv in der 3-Position metalliert und anschließend mit einer Verbindung der Formel III 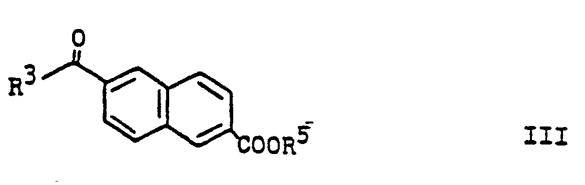 worin R³ die zur Formel genannte Bedeutung hat und R⁵ C₁-C₈-Alkyl bedeutet, umsetzt zu einer Verbindung der Formel I, worin R² Hydroxy und Y die Gruppe COOR⁵ mit R⁵ = C₁-C₈-Alkyl darstellt,
α) gegebenenfalls die erhaltene Verbindung selektiv halogeniert zu einer Verbindung der Formel worin R² Halogen und Y die Gruppe -COOR^{S} mit R⁵ = C₁-C₈-Alkyl bedeutet,
β) gegebenenfalls eine Verbindung der Formel worin R² Halogen und Y die Gruppe -COOR⁵ mit R⁵ = C₁-C₈-Alkyl darstellt, mit einer Verbindung der Formel IV 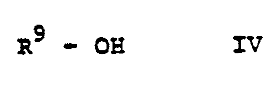 worin R⁹ C₁-C₆-Alkyl oder einen Phenylrest darstellt, der im Kern 1-3fach substituiert sein kann mit Halogen, Trifluormethyl,Alkyl- und/oder Alkoxy mit 1-6 C-Atomen, in Gegenwart einer Base umsetzt zu einer Verbindung der Formel I, worin R² die Gruppe -OR⁹ und Y die Gruppe -COOR⁵ mit R⁵ = C₁-C₈-Alkyl darstellt, oder
b) eine Verbindung der Formel V 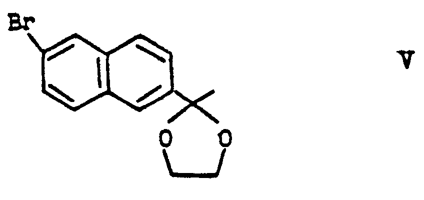 selektiv in der 2-Position metalliert und anschließend mit einer Verbindung der Formel VI 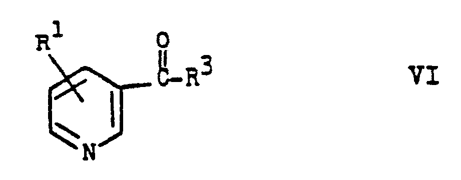 worin R¹ und R³ die zur Formel I angegebenen Bedeutungen haben, zu einer Verbindung der Formel VII 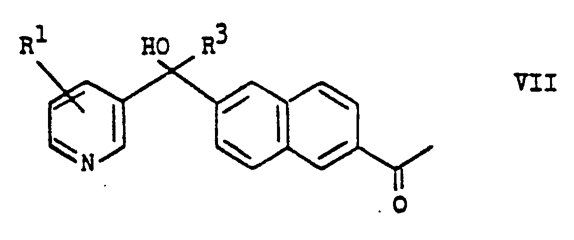 umsetzt, und
i) eine Verbindung der Formel VII, worin R¹ und R³ die zur Formel I angegebene Bedeutung haben, R³ jedoch nicht Wasserstoff ist, oxydiert zu einer Verbindung der Formel I, worin R² die Hydroxy- und Y die Carboxygruppe darstellen,
ii) in einer Verbindung der Formel VII, worin R¹ die zur Formel I angegebene Bedeutung hat und R³ Wasserstoff bedeutet, zunächst die Hydroxygruppe mit einer Schutzgruppe schützt, anschließend die Verbin dung oxidiert und schließlich die Schutzgruppe wieder abspaltet, wobei man zu einer Verbindung der Formel I gelangt, worin R² die Hydroxygruppe, R³ Wasserstoff und Y die Carboxygruppe darstellen,
iii) eine Verbindung der Formel VII, worin R¹ die zur Formel genannte Bedeutung hat und R³ primäres oder sekundäres Alkyl bedeuten, zunächst dehydratisiert und anschließend katalytisch hydriert zu einer Verbindung der Formel VIII 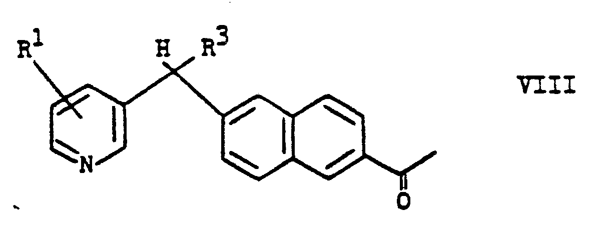 worin R¹ und R³ die oben genannten Bedeutungen haben, oder
eine Verbindung der Formel VII selektiv halogeniert zu einer Verbindung der Formel IX 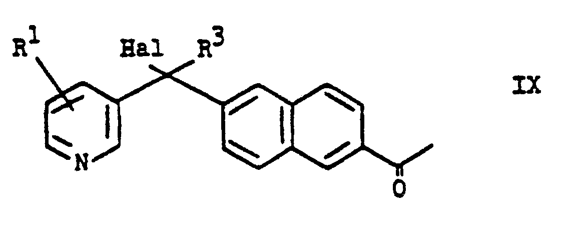 worin R¹ und R³ die zur Formel I genannten Bedeutungen haben und Hal Chlor, Brom oder Jod darstellt, und die erhaltene Verbindung entweder reduziert zu einer Verbindung der Formel VIII 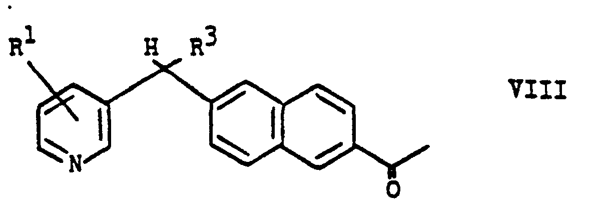 oder mit einer Verbindung der Formel IV 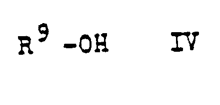 worin R⁹ C₁-C₆-Alkyl oder einen Arylrest darstellt, der im Kern 1-3fach substituiert sein kann mit Halogen, Trifluormethyl Alkyl und/oder Alkoxy mit 1-6 C-Atomen, in Gegenwart einer Base zu einer Verbindung der Formel X 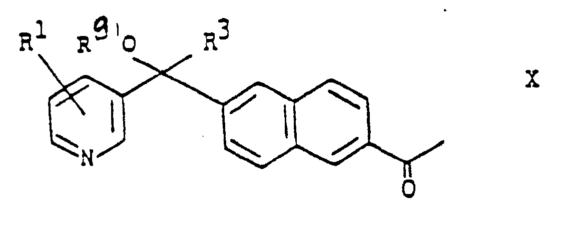 umsetzt, und eine erhaltene Verbindung der Formel VIII, IX oder X oxidiert zu einer Verbindung der Formel I, worin R¹ und R³ die oben zur Formel genannten Bedeutungen haben, Y die Carboxygruppe darstellt und R² Halogen, bzw. die Gruppe OR⁹ bedeutet, und gegebenenfalls eine nach Verfahren a) oder b) erhaltene Verbindung der Formel I, worin Y Carboxyl oder Alkoxycarbonyl darstellt, in die entsprechenden Ester, Amide oder Salze überführt, oder reduziert zu einer Verbindung der Formel I, worin Y die Gruppe -CH₂OH bedeutet, und gegebenenfalls eine erhaltene Verbindung der Formel I in die Säureadditionssalze überführt.

2. Verfahren gemäß Anspruch 1, dadurch gekennzeichnet, daß man eine Verbindung der Formel I, worin
R¹ Wasserstoff oder in 4- oder 5-Stellung Halogen, C₁-C₄-Alkyl oder C₅-C₇-Cycloalkyl,
R² Hydroxy, Halogen, C₁-C₄-Alkoxy, unsubstituiertes Phenoxy oder Phenoxy 1-2-fach substituiert mit Methyl, Ethyl, Methoxy, Ethoxy, Halogen oder Trifluormethyl,
R³ Wasserstoff, C₁-C₆-Alkyl, C₅-C₇-Cycloalkyl, unsubstituiertes Phenyl oder Phenyl, das 1-2fach substituiert ist mit Methyl, Ethyl, Hydroxy, Methoxy, Ethoxy, Halogen oder Trifluormethyl, oder 3-Pyridyl, das in 4- oder 5-Stellung mit Methyl oder Ethyl substituiert sein kann
Y 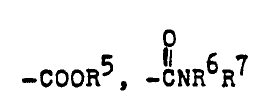 oder CH₂OH, wobei R⁵ Wasserstoff, C₁-C₄-Alkyl, C₂-C₄-Alkenyl, C₅-C₇-Cycloalkyl, Phenethyl, Benzyl oder ein physiologisch verträgliches Metall-, NH₄- oder substituiertes NH₄-lon darstellt,
R⁶ Wasserstoff, C₁-C₄-Alkyl, Cyclopentyl, Cyclohexyl, Phenyl, Benzyl oder Phenethyl darstellt, und R⁷ Wasserstoff oder C₁-C₄-Alkyl, oder R⁶ und R⁷ zusammen eine -(CH₂)ₙ-Gruppe mit n = 4 oder 5 oder eine -CH₂)₂-O-(CH₂)₂-Gruppe darstellen, bedeuten, sowie deren physiologisch verträgliche Säureadditionssalze herstellt.

3. Verfahren gemäß Anspruch 1, dadurch gekennzeichnet, daß man Verbindungen der Formel I, worin R¹ Wasserstoff, C₁-C₂-Alkyl oder Phenyl jeweils in 4-Stellung, R² Hydroxy, C₁-C₂-Alkoxy oder Chlor, R³ Wasserstoff oder C₁-C₂-Alkyl und Y die Gruppe COOR⁵ mit R⁵ = Wasserstoff oder C₁-C₄ bedeuten oder deren physiologisch verträgliche Säureadditionssalze herstellt.

4. Verfahren zur Herstellung eines pharmazeutischen Präparates, dadurch gekennzeichnet, daß man eine Verbindung der Formel gemäß Anspruch 1 oder ein physiologisch verträgliches Säureadditionssalz dieser Verbindung in eine geeignete Darreichungsform überführt.

## Claims (Claims for the following Contracting State(s) : BE CH DE FR GB IT LI LU NL SE)

1. A compound of the formula I 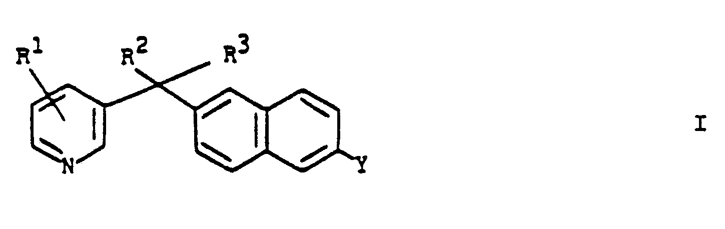 in which:
R' denotes hydrogen or, in the 2-, 4-, 5- or 6-position, halogen, a straight-chain or branched alkyl radical having 1-6 carbon atoms, a cycloalkyl radical having 3-7 carbon atoms, a phenyl-C₁-C₂-alkyl radical or a phenyl radical, each of which can be substituted 1-3 times in the nucleus by halogen, alkyl and/or alkoxy, each having 1-6 carbon atoms, or denotes a hydroxyl radical or an alkoxy radical having 1-6 carbon atoms,
R² denotes a hydroxyl radical, halogen, an alkoxy radical having 1-6 carbon atoms, or a phenoxy radical which can be substituted 1-3 times in the nucleus by halogen, trifluoromethyl, alkyl and/or alkoxy, each having 1-6 carbon atoms,
R³ denotes hydrogen, a cycloalkyl radical having 3-7 carbon atoms, a straight-chain or branched alkyl radical having 1-8 carbon atoms, a straight-chain or branched unsaturated aliphatic hydrocarbon radical having up to 8 carbon atoms and up to 3 double or triple bonds, a phenyl radical which can be substituted 1-3 times in the nucleus by halogen, trifluoromethyl, hydroxyl, alkyl and/or alkoxy, each having 1-6 carbon atoms, or denotes a 2-, 3- or 4-pyridyl radical which can be substituted in the 4-or 5- position by C,-C₄-alkyl,
Y denotes a radical of the formula 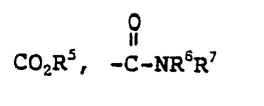 or CH_{z}OH,
R⁵ denoting hydrogen, a straight-chain or branched alkyl radical having up to 8 carbon atoms, a straight-chain or branched unsaturated aliphatic hydrocarbon radical having up to 6 carbon atoms, a cycloaliphatic hydrocarbon radical having 3-7 carbon atoms, an araliphatic hydrocarbon radical having 7-10 carbon atoms, or a physiologically tolerated metal ion, NH4 ion or an ammonium ion which is derived from a primary, secondary or tertiary amine, or a tetraalkyl-ammonium ion,
R⁶ denoting hydrogen, a straight-chain or branched alkyl radical having 1-6 carbon atoms, a cycloaliphatic hydrocarbon radical having 3-7 carbon atoms, an araliphatic hydrocarbon radical which has 7-10 carbon atoms and can be substituted 1-3 times in the nucleus by halogen, trifluoromethyl, alkyl and/or alkoxy, each having 1-6 carbon atoms, or denoting a phenyl radical which can be substituted 1-3 times in the nucleus by halogen, trifluoromethyl, alkyl and/or alkoxy, each having 1-6 carbon atoms,
R⁷ denoting hydrogen, a straight-chain or branched alkyl radical having 1-6 carbon atoms, or
R⁶ and R¹ together denoting a -(CH₂)ₙ- group with n = 3-6, or a -(CH₂)p-Z-(CH₂)p- group with p = ₂ or ₃,
Z being oxygen or N-R⁸,
R⁸ denoting hydrogen or a straight-chain or branched alkyl radical having 1-6 carbon atoms, and its physiologically tolerated acid addition salts.

2. A compound of the formula I as claimed in claim 1, in which
R¹ denotes hydrogen or, in the 4- or 5-position, halogen, C₁-C₄-alkyl or C₅-C₇-cycloalkyl,
R² denotes hydroxyl, halogen, C₁-C₄-alkoxy, unsubstituted phenoxy or phenoxy which is substituted 1-2 times by methyl, ethyl, methoxy, ethoxy, halogen or trifluoromethyl,
R³ denotes hydrogen, C₁-C₈-alkyl, C₅-C₇-cycloalkyl, unsubstituted phenyl or phenyl which is substituted 1-2 times by methyl, ethyl, hydroxyl, methoxy, ethoxy, halogen or trifluoromethyl, or 3-pyridyl which can be substituted in the 4- or 5-position by methyl or ethyl,
Y denotes 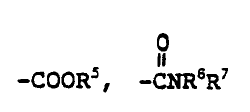 or CH₂0H, R⁵ representing hydrogen, C₁-C₄-alkyl, C₂-C₄-alkenyl, C₅-C₇-cycloalkyl, phenethyl, benzyl or a physiologically tolerated metal ion, NH₄ ion or substituted NH₄ ion,
R⁶ represents hydrogen, C₁-C₄-alkyl, cyclopentyl, cyclohexyl, phenyl, benzyl or phenethyl, and
R⁷ represents hydrogen or C₁-C₄-alkyl, or R⁶ and R⁷ together represent a -(CH₂)ₙ- group with n = 4 or 5, or a (-CH₂)₂-O-(CH₂)₂- group, and its physiologically tolerated acid addition salts.

3. A compound as claimed in claim 1, wherein in formula I R' denotes hydrogen, C₁-C₂-alkyl or phenyl, each in the 4-position, R² denotes hydroxyl, C₁-C₂-alkoxy or chlorine, R³ denotes hydrogen or C₁-C₂- alkyl, and Y denotes the group COOR⁵ with R⁵ denoting hydrogen or C₁-C₄-alkyl.

4. A medicament which contains a compound as claimed in claim 1 together with pharmaceutically customary auxiliaries and vehicles.

5. A compound as claimed in claim 1 for use as a medicament.

6. A process for the preparation of a compound of the formula I as claimed in claim 1 and of its physiologically tolerated acid addition salts, which comprises
a) selective metallation in the 3-position of a compound of the formula II 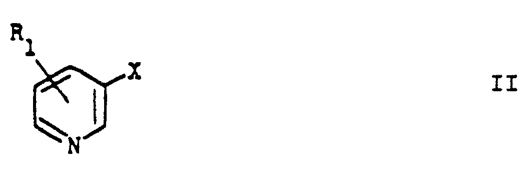 in which
R¹ has the meaning indicated for formula I, and
X denotes chlorine, bromine or iodine,
followed by reaction with a compound of the formula III 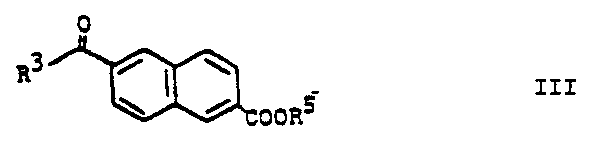 in which
R³ has the meaning mentioned for formula I, and
R⁵ denotes C₁-C₅-alkyl,
to give a compound of the formula I in which R² represents hydroxyl, and Y represents the group COOR⁵ with
R⁵ representing C₁-C₈-alkyl,
α) optionally, selective halogenation of the resulting compound to give a compound of the formula I in which R² denotes halogen, and Y denotes the group -COOR⁵ with R⁵ denoting C₁-C₈-alkyl,
β) optionally, reaction of a compound of the formula I, in which R² represents halogen, and Y represents the group -COOR⁵ with R⁵ representing C₁-C₈-alkyl, with a compound of the formula IV 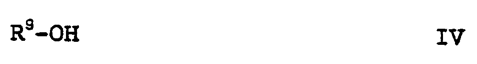 in which
R⁸ represents C₁-C₆-alkyl or a phenyl radical which can be substituted in the nucleus 1-3 times by halogen, trifluoromethyl, alkyl and/or alkoxy having 1-6 carbon atoms, in the presence of a base, to give a compound of the formula I in which R² represents the group -OR⁹, and Y represents the group -COOR^{S} with R⁵ representing C₁-C₈-alkyl, or
b) selective metallation in the 2-position of a compound of the formula V 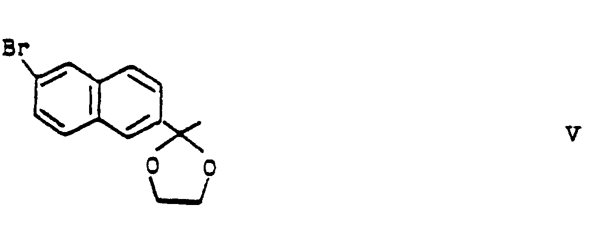 followed by reaction with a compound of the formula VI 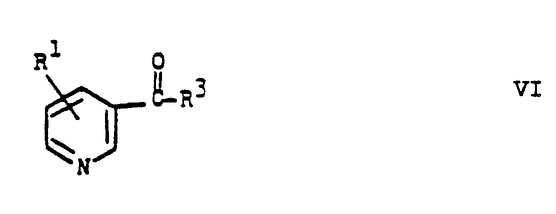 in which
R¹ and R³ have the meanings indicated for formula I, to give a compound of the formula VII 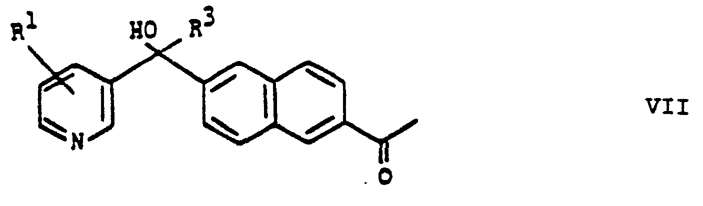 and
i) oxidation of a compound of the formula VII, in which R¹ and R³ have the meaning indicated for formula I, but R³ is not hydrogen, to give a compound of the formula I in which R² represents the hydroxyl group, and Y represents the carboxyl group,
ii) initial protection, with a protective group, of the hydroxyl group in a compound of the formula VII in which R¹ has the meaning indicated for formula I, and R³ denotes hydrogen, then oxidation of the compound and, finally, elimination of the protective group again, thus obtaining a compound of the formula I in which R² represents the hydroxyl group, R³ represents hydrogen, and Y represents the carboxyl group,
iii) initial dehydration followed by catalytic hydrogenation of a compound of the formula VII in which R¹ has the meaning mentioned for formula I, and R³ denotes primary or secondary alkyl, to give a compound of the formula VIII 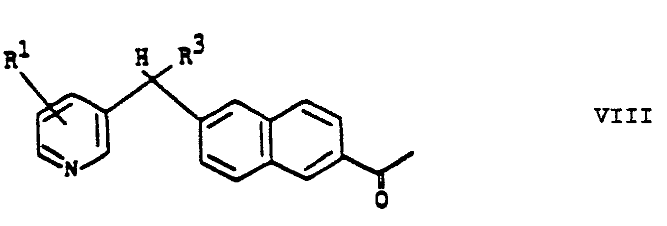 in which
R¹ and R³ have the abovementioned meanings, or selective halogenation of a compound of the formula VII to give a compound of the formula IX 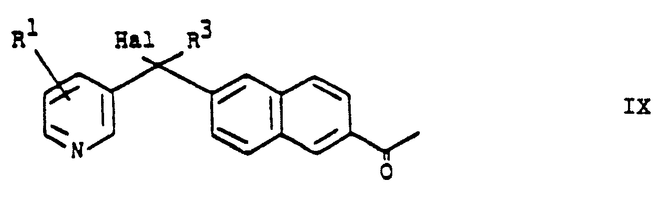 in which
R¹ and R³ have the meanings mentioned for formula I, and
Hal represents chlorine, bromine or iodine,
and either reduction of the resulting compound to give a compound of the formula VIII 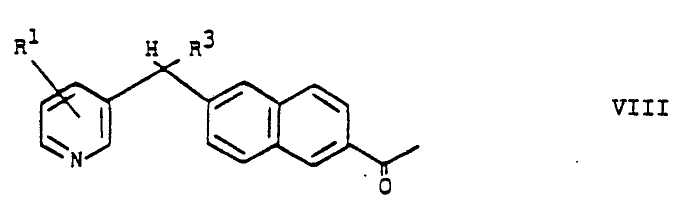 or reaction of the resulting compound with a compound of the formula IV 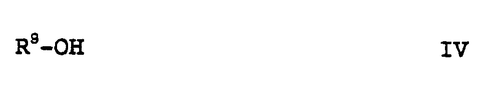 in which
R⁹ represents C₁-C₆-alkyl or an aryl radical which can be substituted in the nucleus 1-3 times by halogen, trifluoromethyl, alkyl and/or alkoxy having 1-6 carbon atoms, in the presence of a base, to give a compound of the formula X 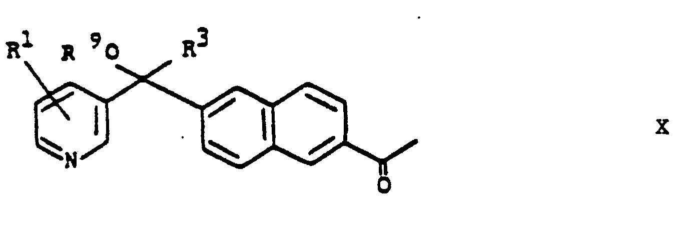 and oxidation of a resulting compound of the formula VIII, IX or X to give a compound of the formula I in which R' and R³ have the meanings mentioned above for formula I, Y represents the carboxyl group, and R² denotes halogen or the group OR⁹, and, optionally, conversion of a compound of the formula I, obtained by process a) or b), in which Y represents carboxyl or alkoxycarbonyl, into the corresponding esters, amides or salts, or reduction to give a compound of the formula I in which Y denotes the group -CH₂OH, and, optionally, conversion of a resulting compound of the formula I into the acid addition salts.

## Claims (Claims for the following Contracting State(s) : AT)

1. A process for the preparation of a compound of the formula I 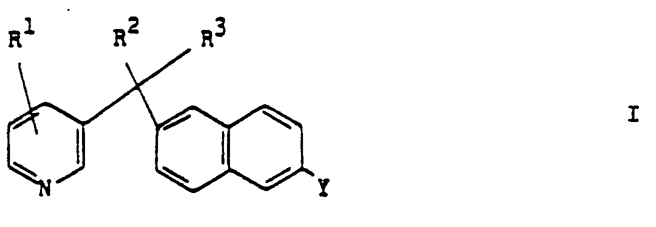 in which:
R¹ denotes hydrogen or, in the 2-, 4-, 5- or 6-position, halogen, a straight-chain or branched alkyl radical having 1-6 carbon atoms, a cycloalkyl radical having 3-7 carbon atoms, a phenyl-C₁-C₂-alkyl radical or a phenyl radical, each of which can be substituted 1-3 times in the nucleus by halogen, alkyl and/or alkoxy, each having 1-6 carbon atoms, or denotes a hydroxyl radical or an alkoxy radical having 1-6 carbon atoms,
R² denotes a hydroxyl radical, halogen, an alkoxy radical having 1-6 carbon atoms, or a phenoxy radical which can be substituted 1-3 times in the nucleus by halogen, trifluoromethyl, alkyl and/or alkoxy, each having 1-6 carbon atoms,
R³ denotes hydrogen, a cycloalkyl radical having 3-7 carbon atoms, a straight-chain or branched alkyl radical having 1-8 carbon atoms, a straight-chain or branched unsaturated aliphatic hydrocarbon radical having up to 8 carbon atoms and up to 3 double or triple bonds, a phenyl radical which can be substituted 1-3 times in the nucleus by halogen, trifluoromethyl, hydroxyl, alkyl and/or alkoxy, each having 1-6 carbon atoms, or denotes a 2-, 3- or 4-pyridyl radical which can be substituted in the 4-or 5- position by C₁-C₄-alkyl,
Y denotes a radical of the formula 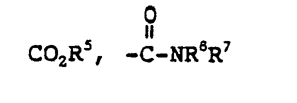 or CH_{z}OH,
R⁵ denoting hydrogen, a straight-chain or branched alkyl radical having up to 8 carbon atoms, a straight-chain or branched unsaturated aliphatic hydrocarbon radical having up to 6 carbon atoms, a cycloaliphatic hydrocarbon radical having 3-7 carbon atoms, an araliphatic hydrocarbon radical having 7-10 carbon atoms, or a physiologically tolerated metal ion, NH₄. ion or an ammonium ion which is derived from a primary, secondary or tertiary amine, or a tetraalkyl-ammonium ion,
R⁶ denoting hydrogen, a straight-chain or branched alkyl radical having 1-6 carbon atoms, a cycloaliphatic hydrocarbon radical having 3-7 carbon atoms, an araliphatic hydrocarbon radical which has 7-10 carbon atoms and can be substituted 1-3 times in the nucleus by halogen, trifluoromethyl, alkyl and/or alkoxy, each having 1-6 carbon atoms, or denoting a phenyl radical which can be substituted 1-3 times in the nucleus by halogen, trifluoromethyl, alkyl and/or alkoxy, each having 1-6 carbon atoms,
R⁷ denoting hydrogen, a straight-chain or branched alkyl radical having 1-6 carbon atoms, or
R and R⁷ together denoting a -(CH₂)ₙ- group with n = 3-6, or a -(CH₂)p-Z-(CH₂)p- group with p = 2 or 3,
Z being oxygen or N-R⁸,
R⁸ denoting hydrogen or a straight-chain or branched alkyl radical having 1-6 carbon atoms, and its physiologically tolerated acid addition salts, which comprises
a) selective metallation in the 3-position of a compound of the formula II 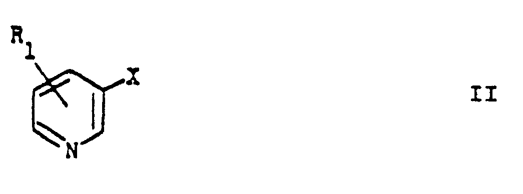 in which
R¹ has the meaning indicated for formula I, and
X denotes chlorine, bromine or iodine,
followed by reaction with a compound of the formula III 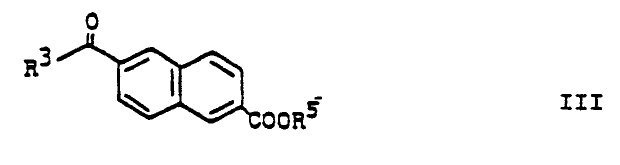 in which
R³ has the meaning mentioned for formula I, and
R⁵ denotes C₁-C₈-alkyl, to give a compound of the formula I in which R² represents hydroxyl, and Y represents the group COOR⁵ with R⁵ representing C₁-C₈-alkyl,
α) optionally, selective halogenation of the resulting compound to give a compound of the formula I in which R² denotes halogen, and Y denotes the group -COOR^{S} with R⁵ denoting C₁-C₈-alkyl,
β) optionally, reaction of a compound of the formula I, in which R² represents halogen, and Y represents the group -COOR^{S} with R⁵ representing C₁-C₈-alkyl, with a compound of the formula IV 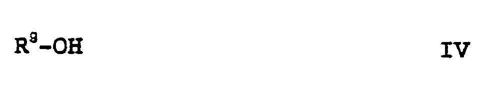 in which
R⁹ represents C₁-C₆-alkyl or a phenyl radical which can be substituted in the nucleus 1-3 times by halogen, trifluoromethyl, alkyl and/or alkoxy having 1-6 carbon atoms, in the presence of a base, to give a compound of the formula I in which R² represents the group - OR⁹, and Y represents the group -COOR⁵ with R⁵ representing C₁-C₈-alkyl, or
b) selective metallation in the 2-position of a compound of the formula V 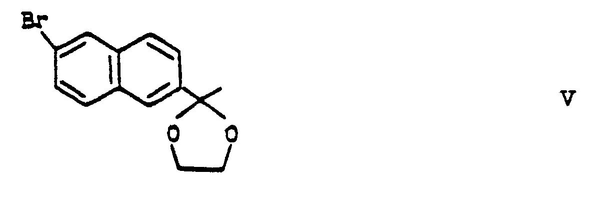 followed by reaction with a compound of the formula VI 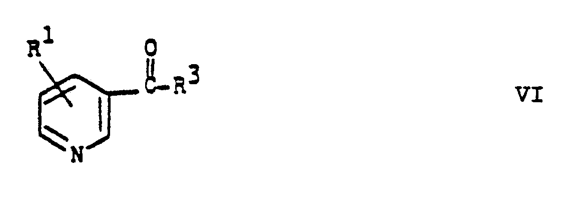 in which
R¹ and R³ have the meanings indicated for formula I, to give a compound of the formula VII 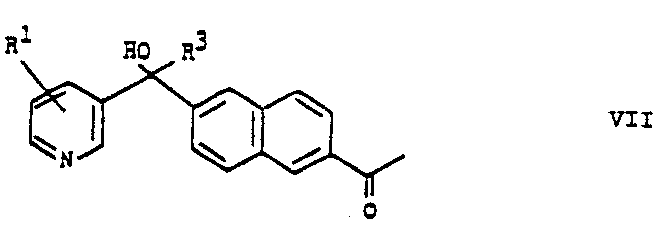 and
i) oxidation of a compound of the formula VII, in which R¹ and R³ have the meaning indicated for formula I, but R³ is not hydrogen, to give a compound of the formula I in which R² represents the hydroxyl group, and Y represents the carboxyl group,
ii) initial protection, with a protective group, of the hydroxyl group in a compound of the formula VII in which R¹ has the meaning indicated for formula I, and R³ denotes hydrogen, then oxidation of the compound and, finally, elimination of the protective group again, thus obtaining a compound of the formula I in which R² represents the hydroxyl group, R³ represents hydrogen, and Y represents the carboxyl group,
iii) initial dehydration followed by catalytic hydrogenation of a compound of the formula VII in which R¹ has the meaning mentioned for formula I, and R³ denotes primary or secondary alkyl, to give a compound of the formula VIII 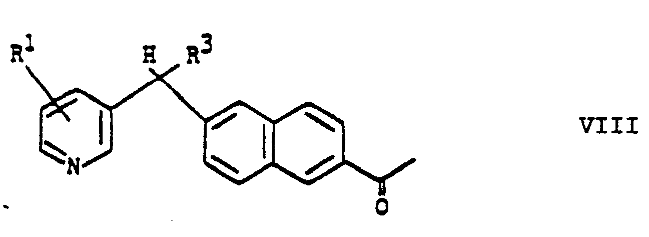 in which
R¹ and R³ have the abovementioned meanings, or selective halogenation of a compound of the formula VII to give a compound of the formula IX 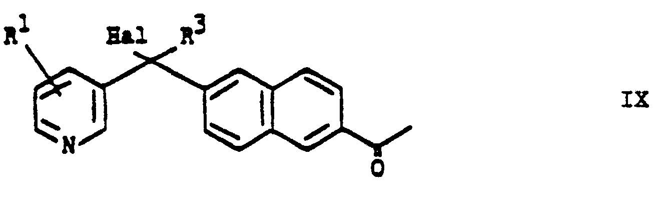 in which
R¹ and R³ have the meanings mentioned for formula I, and
Hal represents chlorine, bromine or iodine, and either reduction of the resulting compound to give a compound of the formula VIII 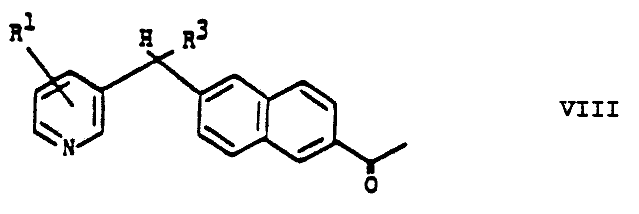 or reaction of the resulting compound with a compound of the formula IV 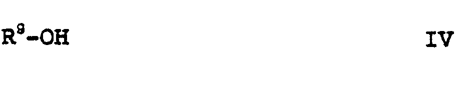 in which
R⁹ represents C₁-C₆-alkyl or an aryl radical which can be substituted in the nucleus 1-3 times by halogen, trifluoromethyl, alkyl and/or alkoxy having 1-6 carbon atoms, in the presence of a base, to give a compound of the formula X 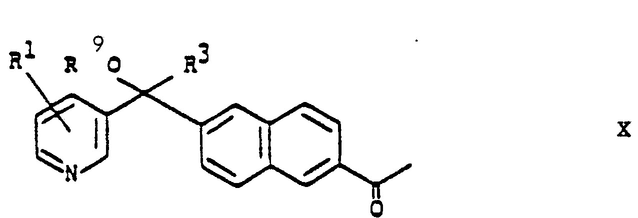 and oxidation of a resulting compound of the formula VIII, IX or X to give a compound of the formula I in which R¹ and R³ have the meanings mentioned above for formula I, Y represents the carboxyl group, and R² denotes halogen or the group OR⁹, and, optionally, conversion of a compound of the formula I, obtained by process a) or b), in which Y represents carboxyl or alkoxycarbonyl, into the corresponding esters, amides or salts, or reduction to give a compound of the formula I in which Y denotes the group -CH₂0H, and, optionally, conversion of a resulting compound of the formula I into the acid addition salts.

2. The process as claimed in claim 1, in which is prepared a compound of the formula I in which
R¹ denotes hydrogen or, in the 4- or 5-position, halogen, C₁-C₄-alkyl or C₅ -C₇-cycloalkyl,
R² denotes hydroxyl, halogen, C₁-C₄-alkoxy, unsubstituted phenoxy or phenoxy which is substituted 1-2 times by methyl, ethyl, methoxy, ethoxy, halogen or trifluoromethyl,
R³ denotes hydrogen, C₁-C₆-alkyl, C₅-C₇-cycloalkyl, unsubstituted phenyl or phenyl which is substituted 1-2 times by methyl, ethyl, hydroxyl, methoxy, ethoxy, halogen or trifluoromethyl, or 3-pyridyl which can be substituted in the 4- or 5-position by methyl or ethyl,
Y denotes 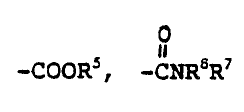 or CH_{z}OH, R⁵ representing hydrogen, C₁-C₄-alkyl, C₂-C₄-alkenyl, C₅-C₇-cycloalkyl, phenethyl, benzyl or a physiologically tolerated metal ion, NH₄ ion or substituted NH₄ ion,
R⁶ represents hydrogen, Ci-C4-alkyl, cyclopentyl, cyclohexyl, phenyl, benzyl or phenethyl, and
R⁷ represents hydrogen or C₁-C₄-alkyl, or R⁶ and R¹ together represent a -(CH2)ₙ- group with n = 4 or 5, or a (-CH₂)₂-O-(CH₂)₂- group, and its physiologically tolerated acid addition salts.

3. The process as claimed in claim 1, wherein a compound of the formula I in which R¹ denotes hydrogen, C₁-C₂-alkyl or phenyl, each in the 4-position, R² denotes hydroxyl, C₁-C₂-alkoxy or chlorine, R³ denotes hydrogen or C₁-C₂-alkyl, and Y denotes the group COOR⁵ with R⁵ denoting hydrogen or C,-C₄-alkyl or its physiologically tolerated acid addition salts are prepared.

4. A process for the preparation of a pharmaceutical product which comprises a compound of the formula I as claimed in claim 1 or a physiologically tolerated acid addition salt of this compound being converted into a suitable formulation.

## Revendications (Revendications pour l'(les) Etat(s) contractant(s) suivant(s) : BE CH DE FR GB IT LI LU NL SE)

1. Composés de formule I 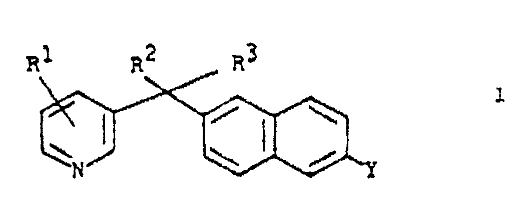 dans laquelle
R' représente un atome d'hydrogène ou en position 2, 4, 5, ou 6: un atome d'halogène, un radical alkyle à chaîne droite ou ramifiée ayant de 1 à 6 atomes de carbone, un radical cycloalkyle ayant de 3 à 7 atomes de carbone, un radical phénylalkyle(C₁-C₂) ou un radical phényle, qui peuvent être substitués de 1 à 3 fois sur le noyau par un ou des atomes d'halogène ou groupes alkyle et/ou alcoxy ayant chacun de 1 à 6 atomes de carbone, ou un radical hydroxy ou alcoxy ayant de 1 à 6 atomes de carbone;
R² représente un atome d'halogène ou un radical hydroxy ou alcoxy ayant de 1 à 6 atomes de carbone, ou un radical phénoxy qui peut être substitué de 1 à 3 fois sur le noyau par un ou des atomes d'halogène ou groupes trifluorométhyle, alkyle et/ou alcoxy ayant chacun de 1 à 6 atomes de carbone;
R³ représente un atome d'hydrogène ou un radical cycloalkyle ayant de 3 à 7 atomes de carbone, un radical alkyle à chaîne droite ou ramifiée ayant de 1 à 8 atomes de carbone, un radical hydrocarboné aliphatique insaturé à chaîne droite ou ramifiée ayant jusqu'à 8 atomes de carbone et comportant jusqu'à 3 doubles ou triples liaisons, un radical phényle qui peut être substitué de 1 à 3 fois sur le noyau par un ou des atomes d'halogène ou groupes trifluorométhyle, hydroxy, alkyle et/ou alcoxy ayant chacun de 1 à 6 atomes de carbone, un radical 2-, 3- ou 4-pyridyle qui peut être substitué en position 4 ou 5 par un groupe alkyle en C₁-C₄;
Y représente un radical de formule 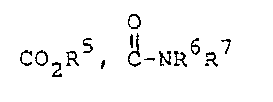 ou CH₂OH;
R⁵ représentant un atome d'hydrogène ou un radical alkyle à chaîne droite ou ramifiée ayant jusqu'à 8 atomes de carbone, un radical hydrocarboné aliphatique insaturé à chaîne droite ou ramifiée ayant jusqu'à 6 atomes de carbone, un radical hydrocarboné cycloaliphatique ayant de 3 à 7 atomes de carbone, un radical hydrocarboné araliphatique ayant de 7 à 10 atomes de carbone ou un ion métallique physiologiquement acceptable, NH₄ ou un ion ammonium qui dérive d'une amine primaire, secondaire ou tertiaire, ou un ion tétraalkylammonium;
R⁶ représentant un atome d'hydrogène ou un radical alkyle à chaîne droite ou ramifiée ayant de 1 à 6 atomes de carbone, un radical hydrocarboné cycloaliphatique ayant de 3 à 7 atomes de carbone, un radical hydrocarboné araliphatique ayant de 7 à 10 atomes de carbone, qui peut être substitué de 1 à 3 fois sur le noyau par un ou des atomes d'halogène ou groupes trifluorométhyle, alkyle et/ou alcoxy ayant chacun de 1 à 6 atomes de carbone, ou un radical phényle qui peut être substitué de 1 à 3 fois sur le noyau par un ou des atome d'halogène ou groupes trifluorométhyle, alkyle et/ou alcoxy ayant chacun de 1 à 6 atomes de carbone;
R⁷ représentant un atome d'hydrogène ou un radical alkyle à chaîne droite ou ramifiée ayant de 1 à 6 atomes de carbone; ou
R⁶ et R⁷ représentent, conjointement, un groupe -(CH2)n avec n = 3-6, ou un groupe -(CH₂)ₚ-Z-(CH₂)_{p̅}avec p = 2 ou 3,
Z représentant un atome d' oxygène ou N -R⁸,
R⁸ représentant un atome d'hydrogène ou un radical alkyle à chaîne droite ou ramifiée ayant de 1 à 6 atomes de carbone, et sels d'addition avec des acides physiologiquement acceptables de ceux-ci.

2. Composés de formule 1 selon la revendication 1, dans lesquels
R¹ représente un atome d'hydrogène ou en position 4 ou 5, un atome d'halogène ou un groupe alkyle en C₁-C₄ ou cycloalkyle en C₅-C₇;
R² représente un atome d'halogène ou un radical hydroxy, alcoxy en C₁-C₄, phénoxy non substitué ou phénoxy substitué 1 ou 2 fois par un ou des atomes d'halogène ou groupes méthyle, éthyle, méthoxy, éthoxy ou trifluorométhyle;
R³ représente un atome d'hydrogène ou un radical alkyle en Ci-Ce, cycloalkyle en C₅-C₇, phényle non substitué ou phényle qui est substitué 1 ou 2 fois par un ou des atomes d'halogène ou groupes méthyle, éthyle, hydroxy, méthoxy, éthoxy ou trifluorométhyle, ou un radical 3-pyridyle qui peut être substitué en position 4 ou 5 par le groupe méthyle ou éthyle;
Y représente 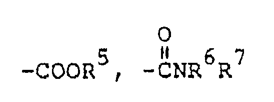 ou CH₂OH,R⁵ représentant un atome d'hydrogène ou un groupe alkyle en C₁-C₄, alcényle en C₂-C₄, cycloalkyle en C₅-C₇, phénéthyle, benzyle ou un ion métallique, NH₄ ou NH₄ substitué, physiologiquement acceptable;
R⁶ représente un atome d'hydrogène ou un groupe alkyle en C₁-C₄, cyclopentyle, cyclohexyle, phényle, benzyle ou phénéthyle, et R⁷ représente un atome d'hydrogène ou en phénéthyle, et R⁷ représente un atome d'hydrogène ou un groupe alkyle en C₁-C₄, ou R⁶ et R⁷ représentent ensemble un groupe -(CH₂)n-avec n = 4 ou 5 ou un groupe -(CH₂)₂-O-(CH₂)₂-, et sels d'addition avec des acides physiologiquement acceptables de ceux-ci.

3. Composés selon la revendication 1, caractérisés en ce que, dans la formule I, R' représente un atome d'hydrogène ou un groupe alkyle en C₁-C₂ ou phényle chacun en position 4, R² représente un atome de chlore ou un groupe hydroxy ou alcoxy en C₁-C₂, R³ représente un atome d'hydrogène ou un groupe alkyle en C₁-C₂ et Y représente un groupe COOR⁵ dans lequel R⁵ représente un atome d'hydrogène ou un radical alkyle en C₁-C₄-.

4. Médicament, caractérisé par une teneur en un composé selon la revendication 1 ainsi qu'en adjuvants et véhicules usuels dans le domaine pharmaceutique.

5. Composé selon la revendication 1, pour utilisation en tant que médicament.

6. Procédé pour la préparation de composés de formule 1 selon la revendication 1, ainsi que de leurs sels d'addition avec des acides physiologiquement acceptables, caractérisé en ce que
a) on soumet à une metallation sélective en position 3 un composé de formule Il 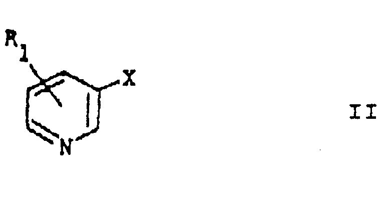 dans laquelle R¹ a la signification indiquée à propos de la formule I et X représente un atome de chlore, brome ou iode, et on le fait ensuite réagir avec un composé de formule III 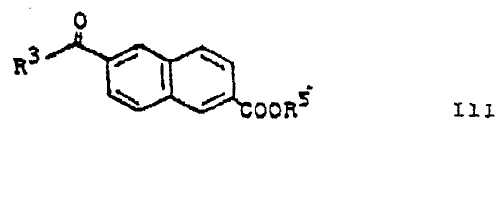 dans laquelle R³ a la signification donnée à propos de la formule I et R⁵ représente un groupe alkyle en C₁-C₈, pour aboutir à un composé de formule I dans lequel R² représente le groupe hydroxy et Y représente un groupe COOR⁵ dans lequel R⁵ représente un groupe alkyle en C₁-C₈,
α) éventuellement, on soumet à une halogénation sélective le composé obtenu pour aboutir à un composé de formule I dans lequel R² représente un atome d'halogène et Y représente le groupe -COOR⁵ et R⁵ représente un groupe alkyle en C,-Cg;
β) éventuellement on fait réagir un composé de formule I dans lequel R² représente un atome d'halogène et Y représente un groupe -COOR^{S} dans lequel R⁵ représente un groupe alkyle en C₁-C₈, avec un composé de formule IV 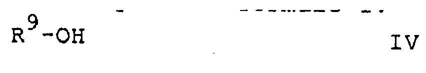 dans laquelle R⁹ représente un radical alkyle en Ci-Ce Ou un radical phényle qui peut être substitué de 1 à 3 fois sur le noyau par un ou des atomes d'halogène ou groupes trifluorométhyle, alkyle et/ou alcoxy ayant de 1 à 6 atomes de carbone, en présence d'une base, pour aboutir à un composé de formule I dans lequel R² représente un groupe -OR⁹ et Y représente un groupe -COOR⁵ dans lequel R⁵ = un reste alkyle en C₁-C₈, ou
b) on soumet à une métallation sélective en position 2 un composé de formule V 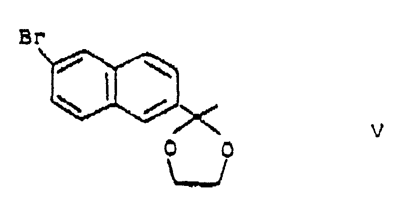 et on le fait ensuite réagir avec un composé de formule VI 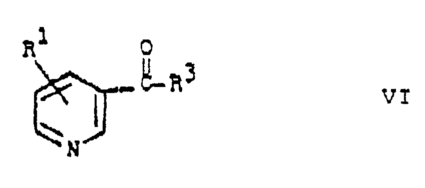 dans laquelle R₁ et R₃ ont les significations indiquées à propos de la formule I, pour aboutir à un compose de formule VII 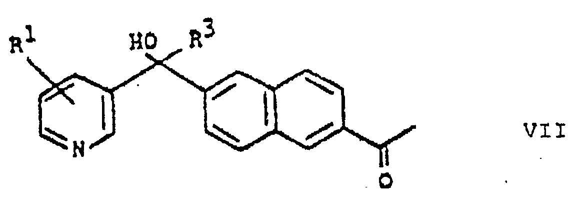 et
i) on oxyde un composé de formule VII dans lequel R' et R³ ont les significations indiquées à propos de la formule I, mais R³ n'est pas un atome d'hydrogène pour aboutir à un composé de formule I dans lequel R² représente le groupe hydroxy et Y représente le groupe carboxy,
ii ) dans un composé de formule VII dans lequel R' a la signification donnée à propos de la formule I et R³ représente un atome d'hydrogène, on protège d'abord le groupe hydroxy au moyen d'un groupe protecteur, ensuite on oxyde le composé et enfin on élimine à nouveau le groupe protecteur, pour aboutir à un composé de formule I dans lequel R² représente le groupe hydroxy, R³ représente un atome d'hydrogène et Y représente le groupe carboxy,
iii) on déshydrate d'abord un composé de formule VII, dans lequel R' a la signification donnée à propos de la formule I et R³ représente un groupe alkyle primaire ou secondaire, et on le soumet ensuite à une hydrogénation catalytique pour aboutir à un composé de formule VIII 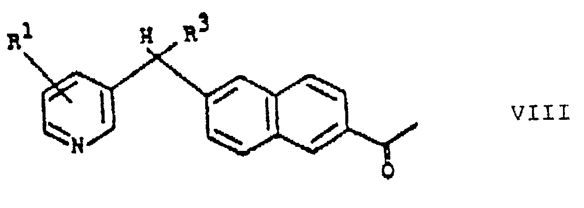 dans laquelle R¹ et R³ ont les significations données plus haut, ou on soumet à une halogénation sélective un composé de formule VII pour aboutir à un composé de formule IX 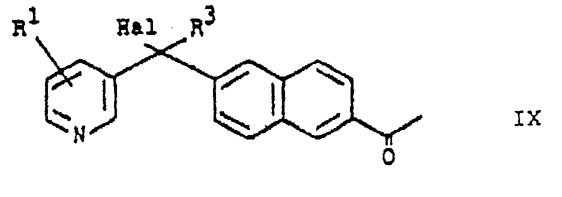 dans laquelle R¹ et R³ ont les significations données à propos de la formule I, et Hal représente le chlore, le brome ou l'iode, et soit on réduit le composé obtenu pour aboutir à un composé de formule VIII 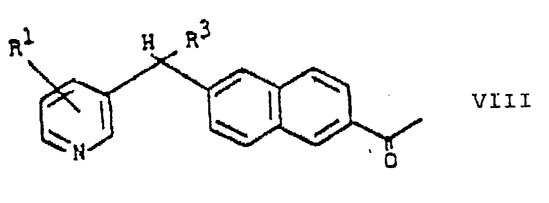 soit on le fait réagir avec un composé de formule IV 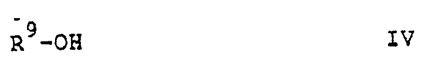 dans laquelle R⁹ représente un radical alkyle en Ci-Cs ou un radical aryle qui peut être substitué de 1 à 3 fois sur le noyau par un ou des atomes d'halogène ou groupes trifluorométhyle, alkyle et/ou alcoxy ayant de 1 à 6 atomes de carbone, en présence d'une base, pour aboutir à un composé de formule X 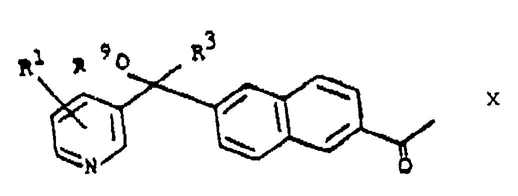 et on oxyde un composé obtenu de formule VIII, IX ou X pour aboutir à un composé de formule 1 dans lequel R¹ et R³ ont les significations données plus haut à propos de la formule I, Y représente le groupe carboxy et R² représente un atome d'halogène ou un groupe OR⁹, et éventuellement on convertit en les esters, amides ou sels correspondants un composé de formule I, obtenu selon le procédé a) ou b), dans lequel Y représente le groupe carboxy ou alcoxycarbony- le, ou on le réduit en un composé de formule I dans lequel Y représente le groupe -CH₂OH, et éventuellement on convertit en les sels d'addition avec des acides un composé de formule 1 obtenu.

## Revendications (Revendications pour l'(les) Etat(s) contractant(s) suivant(s) : suivant: AT)

1. Procédé pour la préparation de composés de formule 1 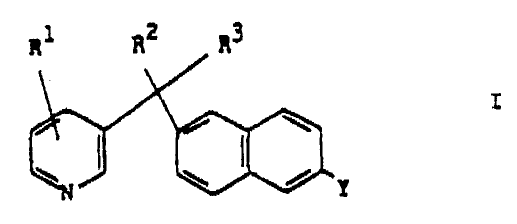 dans laquelle
R¹ représente un atome d'hydrogène ou en position 2, 4, 5 ou 6, un atome d'halogène, un radical alkyle à chaîne droite ou ramifiée ayant de 1 à 6 atomes de carbone, un radical cycloalkyle ayant de 3 à 7 atomes de carbone, un radical phénylalkyle (C₁-C₂) ou un radical phényle, qui peuvent être substitués de 1 à 3 fois sur le noyau par un ou des atome d'halogène ou groupes alkyle et/ou alcoxy ayant chacun de 1 à 6 atomes de carbone, ou un radical hydroxy ou alcoxy ayant de 1 à 6 atomes de carbone;
R² représente un atome d'halogène ou un radical hydroxy ou alcoxy ayant de 1 à 6 atomes de carbone, ou un radical phénoxy qui peut être substitué de 1 à 3 fois sur le noyau par un ou des atomes d'halogène ou groupes trifluorométhyle, alkyle et/ou alcoxy ayant chacun de 1 à 6 atomes de carbone;
R³ représente un atome d'hydrogène ou un radical cycloalkyle ayant de 3 à 7 atomes de carbone, un radical alkyle à chaîne droite ou ramifiée ayant de 1 à 8 atomes de carbone, un radical hydrocarboné aliphatique insaturé à chaîne droite ou ramifiée ayant jusqu'à 8 atomes de carbone et comportant jusqu'à 3 doubles ou triples liaisons, un radical phényle qui peut être substitué de 1 à 3 fois sur le noyau par un ou des atomes d'halogène ou groupes trifluorométhyle, hydroxy, alkyle et/ou alcoxy ayant chacun de 1 à 6 atomes de carbone, un radical 2-, 3- ou 4-pyridyle qui peut être substitué en position 4 ou 5 par un groupe alkyle en C₁-C₄;
Y représente un radical de formule 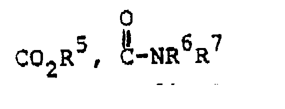 ou CH₂0H;
R⁵ représentant un atome d'hydrogène ou un radical alkyle à chaîne droite ou ramifiée ayant jusqu'à 8 atomes de carbone, un radical hydrocarboné aliphatique insaturé à chaîne droite ou ramifiée ayant jusqu'à 6 atomes de carbone, un radical hydrocarboné cycloaliphatique ayant de 3 à 7 atomes de carbone, un radical hydrocarboné araliphatique ayant de 7 à 10 atomes de carbone ou un ion métallique physiologiquement acceptable, NH₄ ou un ion ammonium qui dérive d'une amine primaire, secondaire ou tertiaire, ou un ion tétraalkylammonium;
R⁶ représentant un atome d'hydrogène ou un radical alkyle à chaîne droite ou ramifiée ayant de 1 à 6 atomes de carbone, un radical hydrocarboné cycloaliphatique ayant de 3 à 7 atomes de carbone, un radical hydrocarboné araliphatique ayant de 7 à 10 atomes de carbone, qui peut être substitué de 1 à 3 fois sur le noyau par un ou des atomes d'halogène ou groupes trifluorométhyle, alkyle et/ou alcoxy ayant chacun de 1 à 6 atomes de carbone, ou un radical phényle qui peut être substitué de 1 à 3 fois sur le noyau par un ou des atomes d'halogène ou groupes trifluorométhyle, alkyle et/ou alcoxy ayant chacun de 1 à 6 atomes de carbone;
R⁷ représentant un atome d'hydrogène ou un radical alkyle à chaîne droite ou ramifiée ayant de 1 à 6 atomes de carbone; ou
R⁶ et R⁷ représentent, conjointement, un groupe -(CH₂)_{n̅} avec n = 3-6, ou un groupe -(CH₂)ₚ-Z-(CH2)_{p̅} avec p = 2 ou 3,
Z représentant un atome d'oxygène ou N-R⁸,
R⁸ représentant un atome d'hydrogène ou un radical alkyle à chaîne droite ou ramifiée ayant de 1 à 6 atomes de carbone,
ainsi que de leurs sels d'addition avec des acides physiologiqument acceptables,
caractérisé en ce que
a) on soumet à une métallation sélective en position 3 un composé de formule Il 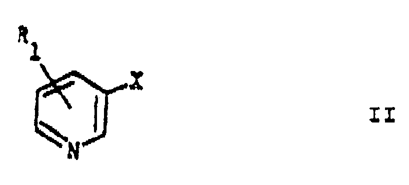 dans laquelle R¹ a la signification indiquée à propos de la formule 1 et X représente un atome de chlore, brome ou iode, et on le fait ensuite réagir avec un composé de formule III 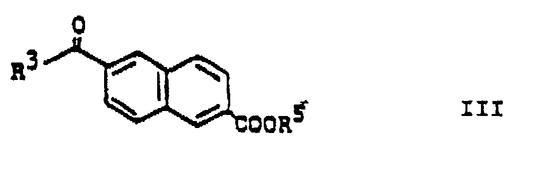 dans laquelle R³ a la signification donnée à propos de la formule I et R⁵ représente un groupe alkyle en C₁-C₈, pour aboutir à un composé de formule I dans lequel R² représente le groupe hydroxy et Y représente un groupe COOR⁵ dans lequel R⁵ représente un groupe alkyle en Ci-Cₛ,
a) éventuellement, on soumet à une halogénation sélective le composé obtenu, pour aboutir à un composé de formule I dans lequel R² représente un atome d'halogène et Y représente le groupe -COOR^{S} et R⁵ représente un groupe alkyle en C₁-C₈;
β) éventuellement on fait réagir un composé de formule I dans lequel R² représente un atome d'halogène et Y représente un groupe -COOR⁵ dans lequel R⁵ représente un groupe alkyle en C₁-C₈, avec un composé de formule IV 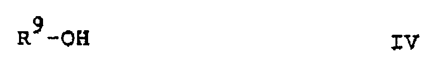 dans laquelle R⁹ représente un radical alkyle en Ci-Ce ou un radical phényle qui peut être substitué de 1 à 3 fois sur le noyau par un ou des atomes d'halogène ou groupes trifluorométhyle, alkyle et/ou alcoxy ayant de 1 à 6 atomes de carbone, en présence d'une base, pour aboutir à un composé de formule I dans lequel R² représente un groupe -OR⁹ et Y représente un groupe -COOR^{S} dans lequel R⁵ = un reste alkyle en C₁-C₈, ou
b) on soumet à une métallation sélective en position 2 un composé de formule V 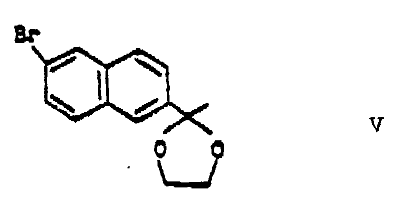 et on le fait ensuite réagir avec un composé de formule VI 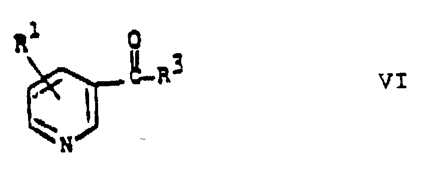 dans laquelle R₁ et R₃ ont les significations indiquées à propos de la formule I, pour aboutir à un composé de formule VII 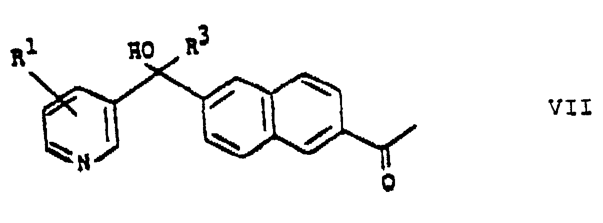 et
i) on oxyde un composé de formule VII dans lequel R¹ et R³ ont les significations indiquées à propos de la formule I, mais R³ n'est pas un atome d'hydrogène pour aboutir à un composé de formule I dans lequel R² représente le groupe hydroxy et Y représente le groupe carboxy,
ii) dans un composé de formule VII dans lequel R¹ a la signification donnée à propos de la formule I et R³ représente un atome d'hydrogène, on protège d'abord le groupe hydroxy ou moyen d'un groupe protecteur, ensuite on oxyde le composé et enfin on élimine à nouveau le groupe protecteur, pour aboutir à un composé de formule I dans lequel R² représente le groupe hydroxy, R³ représente un atome d'hydrogène et Y représente le groupe carboxy,
iii) on déshydrate d'abord un composé de formule VII, dans lequel R¹ a la signification donnée à propos de la formule I et R³ représente un groupe alkyle primaire ou secondaire, et on le soumet ensuite à une hydrogénation catalytique pour aboutir à un composé de formule VIII 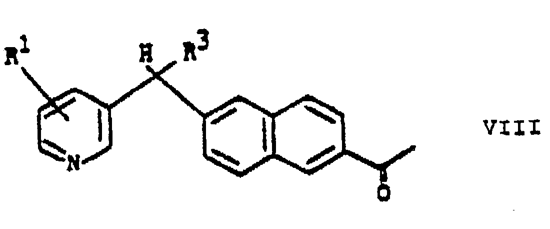 dans laquelle R¹ et R³ ont les significations données plus haut, ou on soumet à une halogénation sélective un composé de formule VII pour aboutir à un composé de formule IX 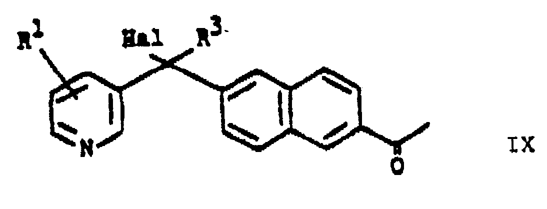 dans laquelle R' et R³ ont les significations données à propos de la formule I, et Hal représente le chlore, le brome ou l'iode, et soit on réduit le composé obtenu pour aboutir à un composé de formule Vlll 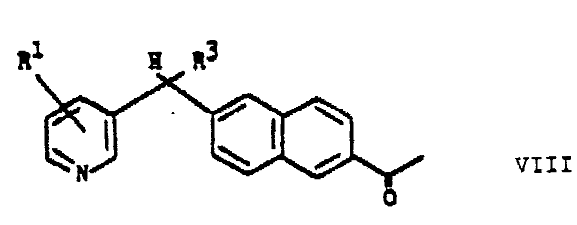 soit on le fait réagir avec un composé de formule IV 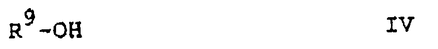 dans laquelle R⁹ représente un radical alkyle en Ci-Ce ou un radical aryle qui peut être substitué de 1 à 3 fois sur le noyau par un ou des atomes d'halogène ou groupes trifluorométhyle, alkyle et/ou alcoxy ayant de 1 à 6 atomes de carbone, en présence d'une base, pour aboutir à un composé de formule X 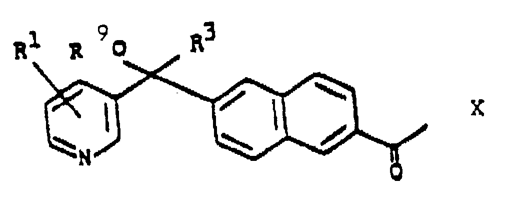 et on oxyde un composé obtenu de formule VIII, IX ou X pour aboutir à un composé de formule I dans lequel R¹ et R³ ont les significations données plus haut à propos de la formule I, Y représente le groupe carboxy et R² représente un atome d'halogène ou un groupe OR⁹, et éventuellement on convertit en les esters, amides ou sels correspondants un composé de formule I, obtenu selon le procédé a) ou b), dans lequel Y représente le groupe carboxy ou alcoxycarbony- le, ou on le réduit en un composé de formule I dans lequel Y représente le groupe -CH₂0H, et éventuellement on convertit en les sels d'addition avec des acides un composé de formule 1 obtenu.

2. Procédé selon la revendication 1, caractérisé en ce que l'on prépare un composé de formule I dans lequel
R¹ représente un atome d'hydrogène ou en position 4 ou 5, un atome d'halogène, ou un groupe alkyle en C₁-C₄ ou cyclo alkyle en C₅-C₇;
R² représente un atome d'halogène ou un radical hydroxy, alcoxy en C₁-C₄, phénoxy non substitué ou phénoxy substitué 1 ou 2 fois par un ou des atomes d'halogène ou groupes méthyle, éthyle, méthoxy, éthoxy ou trifluorométhyle;
R³ représente un atome d'hydrogène ou un radical alkyle en Ci-Ce, cycloalkyle en C₅-C₇, phényle non substitué ou phényle qui est substitué 1 ou 2 fois par un ou des atomes d'halogène ou groupes méthyle, éthyle, hydroxy, méthoxy, éthoxy ou trifluorométhyle, ou un radical 3-pyridyle qui peut être substitué en position 4 ou 5 par le groupe méthyle ou 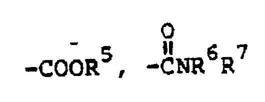 éthyle;
Y représente ou CH_{z}OH, R⁵ représentant un atome d'hydrogène ou un groupe alkyle en C₁-C₄, alcényle en C₂-C₄, cycloalkyle en C₅-C₇, phénéthyle, benzyle ou un ion métallique, NH4 ou NH4 substitué, physiologiquement acceptable;
R⁶ représente un atome d'hydrogène ou un groupe alkyle en C₁-C₄, cyclopentyle, cyclohexyle, phényle, benzyle ou phénéthyle, et R⁷ représente un atome d'hydrogène ou un groupe alkyle en C₁-C₄, ou R⁶ et R⁷ représentent ensemble un groupe (-CH₂)ₙ- avec n = 4 ou 5 ou un groupe -(CH₂)₂-O-(CH₂)₂-,
ainsi que ses sels d'addition avec des acides physiologiquement acceptables.

3. Procédé selon la revendication 1, caractérisé en ce que l'on prépare des composés de formule 1 dans lesquels R¹ représente un atome d'hydrogène ou un groupe alkyle en C₁-C₂ ou phényle chacun en position 4, R² représente un atome de chlore ou un groupe hydroxy ou alcoxy en C₁-C₂, R³ représente un atome d'hydrogène ou un groupe alkyle en C₁-C₂ et Y représente un groupe COOR⁵ dans lequel R⁵ représente un atome d'hydrogène ou un radical alkyle en C₁-C₄, ou leurs sels d'addition avec des acides physiologiquement acceptables.

4. Procédé pour la préparation d'une composition pharmaceutique, caractérisé en ce que l'on met sous une forme pharmaceutique appropriée un composé de formule I selon la revendication 1 ou un sel d'addition avec un acide physiologiquement acceptable de ce composé.
